(19)

Europäisches
Patentamt
European
Patent Office
Office européen
des brevets

(11) **EP 2 905 631 A1**

(12) **EUROPEAN PATENT APPLICATION**

(43) Date of publication:
**12.08.2015 Bulletin 2015/33**

(51) Int Cl.:
*G01R 33/28* (2006.01)      *G01R 33/34* (2006.01)
*G01R 33/341* (2006.01)      *G01R 33/38* (2006.01)
*G01R 33/465* (2006.01)      *A61B 5/055* (2006.01)
*A61B 5/00* (2006.01)      *G01R 33/36* (2006.01)

(21) Application number: **14191760.9**

(22) Date of filing: **19.12.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **21.12.2007 US 8646**
**21.12.2007 US 8669**
**14.05.2008 US 127514**

(62) Document number(s) of the earlier application(s) in accordance with Art. 76 EPC:
**08866830.6 / 2 223 133**

(71) Applicant: **T2 Biosystems, Inc.**
**Cambridge MA 02141-1920 (US)**

(72) Inventors:
• **Lowery, Thomas J.**
**Belmont, MA 02478 (US)**
• **Prado, Pablo J.**
**Boston, MA 02109 (US)**

(74) Representative: **Bassil, Nicholas Charles et al**
**Kilburn & Strode LLP**
**20 Red Lion Street**
**London WC1R 4PJ (GB)**

(54) **Magnetic resonance system with implantable components and methods of use thereof**

(57)      Nuclear magnetic resonance systems and methods of use thereof are provided. The systems employ implantable radiofrequency coils (105) and optionally implantable magnets (101). The systems can employ weak permanent magnets and/or permanent magnets that provide magnetic fields that are much less homogeneous than in conventional systems. This allows, for example, for inexpensive and simple probeheads for nuclear magnetic resonance relaxometry with suitable biosensors. The methods of the present invention allow *in vivo* magnetic resonance measurements and, in particular, monitoring of analytes and determination of medical diagnostic information, for example, based on determined magnetic resonance parameters.

FIG. 2A

**(Cont. next page)**

FIG. 2B

**Description**

RELATED APPLICATIONS

This application claims priority to and the benefit of U.S. Provisional Application No. 61/008,646, filed December 21, 2007; and this application claims priority to and the benefit of U.S. Provisional Application No. 61/008,669, filed December 21, 2007; and this application claims priority to and the benefit of U.S. Provisional Application No. 61/127,514, filed May 14, 2008. The entire contents of the prior applications are incorporated herein by reference in their entirety.

BACKGROUND OF THE INVENTION

Current diagnostic systems involve, for example, microarray technology, polymerase chain reaction (PCR), in situ hybridization, antibody-based immunoassays (e.g. enzyme-linked immunosorbant assays), chemiluminescence, nephelometry, and/or photometry. Generally, these systems cannot perform the diversity of assays at high sensitivity that is possible with an NMR-based nanosensor system.

Nuclear magnetic resonance (NMR) systems make use of nuclear magnetic resonance of atomic nuclei contained in a sample and are known to be able to provide a large variety of information characterizing a sample and corresponding sample components. Systems include, for example, magnetic resonance imaging (MRI) devices, magnet resonance spectrometers and magnetic resonance relaxometers. The nature of nuclear magnetic resonance phenomenon requires the presence of a magnetic field upon excitation with a radiofrequency electromagnetic wave. Thus, generally, NMR systems include a magnet and a radiofrequency coil, either as separate system components or combined in a probehead.

Magnets that are typically preferred in magnetic resonance systems provide magnetic fields with high magnetic field strength and high homogeneity. Magnets known to satisfy such requirements are usually large and/or expensive. They are therefore not suitable for portable devices and/or implantation devices, and/or not suitable as part of disposable probeheads. Thus, a need exists for small, inexpensive probeheads for use in magnetic resonance systems, allowing portability, implantation and/or one-time use applications.

Magnetic nanosensors are derivatized superparamagnetic nanoparticles that form clusters (aggregates) or nanoassemblies as a function of the presence or concentration of their intended molecular target. It is thought that when superparamagnetic nanoparticles assemble into clusters and the effective cross sectional area becomes larger, a nanoassembly becomes more efficient at dephasing the spins of surrounding water (or other solvent) protons, leading to a measurable change of the relaxation rates (1/T2). Examples of magnetic nanosensors are described, for example, in Perez et al., "Use of Magnetic Nanoparticles as Nanosensors to Probe for Molecular Interactions," Chem Bio Chem, 2004, 5, 261-264, and in U.S. Patent Application Publication No. US2003/0092029 (Josephson et al.).

Provided magnetic resonance systems and methods of the present invention address some of the limitations in the art. Provided implantable systems and methods are particularly suitable but not limited to magnetic relaxation measurements, because relaxation measurements require less homogenous fields.

SUMMARY OF THE INVENTION

One embodiment of the present invention is a nuclear magnetic resonance system for measuring magnetic resonance signals from a sample contained in a sample volume *in-vivo*. The system comprises a magnet or magnetic field generator positioned to provide a magnetic field in a sample volume, the magnetic field being suitable to allow measuring magnetic resonance signal; a probehead suitable for partial or complete implantation in a subject, the probehead comprising a radiofrequency circuit that includes a radiofrequency coil wound to form a space capable of accommodating a sample volume and a port, the port allowing a sample to enter the sample volume, wherein the sample volume contains magnetic particles and the port is adapted to allow an analyte to enter the sample volume and to prevent, partly or completely, the magnetic particles from leaving the sample volume; an external coil for disposition outside the subject's body, wherein the external coil is suitable for inductive coupling to the radiofrequency circuit to form a radiofrequency resonant circuit; and a control unit for disposition outside the subject's body, wherein the control unit is connected to the external coil and comprises logic circuitry to control the radiofrequency resonant circuit and allows acquisition and processing of magnetic resonance signals received by the radiofrequency resonant circuit.

Another embodiment of the present invention is nuclear magnetic resonance system for measuring magnetic resonance relaxation signals from a sample contained in a sample volume *in-vivo*. The system comprises: a single-sided permanent magnet or magnetic field generator for disposition outside a subject's body and near a sample volume to provide a magnetic field in the sample volume, the magnetic field being suitable to allow measuring magnetic resonance relaxation signals; a probehead suitable for partial or complete implantation in a subject, the probehead comprising a radiofrequency circuit that includes a radiofrequency coil wound to form a space capable of accommodating a sample volume and a port, the port allowing a sample to enter the sample volume; an external coil for disposition outside the subject's body, wherein the external coil is suitable for inductive coupling to the radiofrequency circuit to form a radiofrequency resonant circuit; and a control unit for disposition outside the subject's body, wherein the control unit is connected to the external coil and comprises logic circuitry to control the radiofrequency resonant circuit and allows acquisition and processing of magnetic resonance relaxation signals received by the radiofrequency resonant circuit.

Yet another embodiment of the invention provides a nuclear magnetic resonance system for measuring magnetic resonance relaxation signals from a sample contained in a sample volume *in-vivo.* The system comprises: a probehead suitable for partial or complete implantation in a subject, the probehead comprising a radiofrequency circuit that includes a capacitor and a radiofrequency coil wound to form a space capable of accommodating a sample volume and a port, the port allowing a sample to enter the sample volume; and a permanent magnet positioned near or around the radiofrequency coil to provide a magnetic field in the sample volume, the magnetic field being suitable to allow measuring magnetic resonance relaxation signal; an external coil for disposition outside the subject's body, wherein the external coil is suitable for inductive coupling to the radiofrequency circuit to form a radiofrequency resonant circuit; and a control unit for disposition outside the subject's body, wherein the control unit is connected to the external coil and comprises logic circuitry to control the radiofrequency resonant circuit and allows acquisition and processing of magnetic resonance relaxation signals received by the radiofrequency resonant circuit.

Still another embodiment of the present invention is nuclear magnetic resonance system for measuring magnetic resonance relaxation signals from a sample contained in a sample volume *in-vivo.* The system comprises: a single-sided permanent magnet or magnetic field generator for disposition outside a subject's body and near a sample volume to provide a magnetic field in the sample volume, the magnetic field being suitable to allow measuring magnetic resonance relaxation signals; a probehead suitable for partial or complete implantation in a subject, the probehead comprising a radiofrequency circuit that includes a radiofrequency coil wound to form a space capable of accommodating a sample volume and a port, the port allowing a sample to enter the sample volume, wherein the sample volume contains at least one sensor particle and the port is adapted to allow an analyte to enter the sample volume and to prevent, partly or completely, the sensor particles from leaving the sample volume; an external coil for disposition outside the subject's body, wherein the external coil is suitable for inductive coupling to the radiofrequency circuit to form a radiofrequency resonant circuit; and a control unit for disposition outside the subject's body, wherein the control unit is connected to the external coil and comprises logic circuitry to control the radiofrequency resonant circuit and allows acquisition and processing of magnetic resonance relaxation signals received by the radiofrequency resonant circuit.

Yet another embodiment of the invention provides a nuclear magnetic resonance system for measuring magnetic resonance relaxation signals from a sample contained in a sample volume *in-vivo.* The system comprises: a probehead suitable for partial or complete implantation in a subject, the probehead comprising a radiofrequency circuit that includes a capacitor and a radiofrequency coil wound to form a space capable of accommodating a sample volume and a port, the port allowing a sample to enter the sample volume, wherein the sample volume contains at least one sensor particle and the port is adapted to allow an analyte to enter the sample volume and to prevent, partly or completely, the sensor particles from leaving the sample volume; and a permanent magnet positioned near or around the radiofrequency coil to provide a magnetic field in the sample volume, the magnetic field being suitable to allow measuring magnetic resonance relaxation signal; an external coil for disposition outside the subject's body, wherein the external coil is suitable for inductive coupling to the radiofrequency circuit to form a radiofrequency resonant circuit; and a control unit for disposition outside the subject's body, wherein the control unit is connected to the external coil and comprises logic circuitry to control the radiofrequency resonant circuit and allows acquisition and processing of magnetic resonance relaxation signals received by the radiofrequency resonant circuit.

Still other embodiments include methods for determining a magnetic resonance relaxation parameter associated with a sample contained in a sample volume *in-vivo in* a subject using a nuclear magnetic resonance system. In certain embodiments, the method comprises: positioning a magnet or magnetic field generator of the nuclear magnetic resonance system near or around the sample to provide a magnetic field in the sample suitable to allow measuring magnetic resonance signals; implanting partially or completely a probehead of the nuclear magnetic resonance system within a subject's body, the probehead comprising: a radiofrequency circuit that includes a radiofrequency coil wound to form a space capable of accommodating a sample volume and a port, the port allowing a sample to enter the sample volume, wherein the sample volume contains at least one sensor particle and the port is adapted to allow an analyte to enter the sample volume and to prevent, partly or completely, the one or more sensor particles from leaving the sample volume, and at least part the sensor particles aggregate in the presence of analyte to change a magnetic resonance signal of a sample; positioning an external coil outside the subject's body, wherein the external coil is inductively coupled to the radiofrequency circuit to form a radiofrequency resonant circuit; controlling with a control unit positioned outside the subject's body the radiofrequency circuit to apply the radiofrequency pulse or pulse sequence to the sample volume in the presence of the magnetic field; and acquiring and processing part or effectively all of the magnetic resonance signals from the sample in the sample volume sensed by the radiofrequency resonant circuit to determine a magnetic resonance relaxation parameter.

Additional embodiments provide methods of monitoring analytes in a body of a patient. In certain embodiments the method comprises: implanting an implantable diagnostic device at an exposed treatment site; and detecting or measuring the presence and/or concentration of one or more analytes using magnetic resonance measurement.

In another embodiment, the present invention provides a method of determining organ transplant rejection or acceptance in a patient. The method comprises: implanting an implantable magnetic resonance diagnostic device in a patient having undergone or undergoing an organ transplant surgery, the implanted diagnostic device detecting or measuring the presence and/or concentration of one or more analytes; and monitoring the one or more analytes in response to the organ transplant using the output of the implanted diagnostic device; wherein the output of the implanted device conveys information indicating whether an organ transplant is being rejected or accepted in the patient.

The foregoing will be apparent from the following more particular description of example embodiments of the invention, as illustrated in the accompanying drawings in which like reference characters refer to the same parts throughout the different views. The drawings are not necessarily to scale, emphasis instead being placed upon illustrating embodiments of the present invention.

## BRIEF DESCRIPTION OF THE DRAWINGS

FIG. 1 provides a schematic representation of two nuclear magnetic resonance systems with probeheads implanted in mammalian body, one (system A) employing a probehead with a double sided magnet and the other (system B) a probehead without a magnet but an external one-sided magnet.

FIG. 2 provides a schematic representation of two nuclear magnetic resonance (NMR) systems with probeheads implanted in mouse, one (system A) employing a probehead with a double sided magnet and the other (system B) a probehead without a magnet but an external one-sided magnet.

FIG. 3 presents two schematic views (a top view and a side view) of a probehead that is suitable for the systems of the present invention, the probehead including a radiofrequency coil surrounding by a double-sided magnet.

FIG. 4 presents two schematic views (a top view and a side view) of a probehead that is suitable for the systems of the present invention, the probehead including a planar radiofrequency coil adjacent to a magnet.

FIG. 5 shows a probehead in a corresponding pickup assembly for connection to a spectrometer.

FIG.6 provides a magnitude spectrum obtained with the device of FIG. 5.

FIG. 7 provides a $T_2$ relaxation curve obtained with the device of FIG. 5.

## DETAILED DESCRIPTION OF CERTAIN EMBODIMENTS OF THE INVENTION

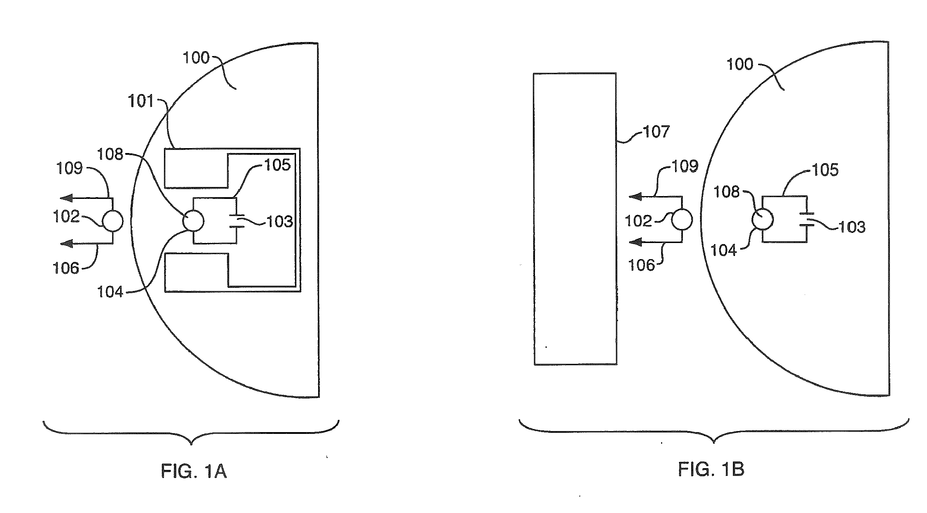

One aspect of the present invention is an implantable device for *in vivo* Magnetic Resonance (MR) sensing. It allows measuring MR signals from a selected volume inside the body of an animal. Sensing is performed by using an apparatus configuration where components of a device include a resonant radio-frequency (RF) circuit (see Figure 1-A) or a resonant RF circuit and a small magnet (see Figure 1-B) implanted in the body of a subject (e.g., an animal), wherein an RF spectrometer is not included in the implantable components. A static magnetic field needed for an MR sensing method is generated by either a small implanted permanent magnet (e.g., a magnet implanted in the proximity of or around an implanted RF coil) (Fig. A), or by an external magnet (Fig. B). An external magnet, e.g., a portable open permanent magnet, can be placed at an external location of a subject close to the location of an implanted device for generation of a magnetic field. An implanted RF coil, when part of an RF resonant circuit, is electromagnetically (inductively, non contact) coupled to an external coil. MR measurements are controlled with an external spectrometer, connected to the external coil. Provided configurations allow measuring signals from a specific location inside the body and may also allow monitoring properties of body fluids and/or tissues by their interaction with a small biological or chemical sensor particle (e.g., a magnetic particle) implanted together with an MR device.

Another aspect of the present invention is a means for obtaining magnetic resonance relaxation measurements from an implanted radiofrequency (RF) detection coil, wherein the means includes an external RF coil and instrument that is not physically attached to the detection coil (e.g., inductive coupling of an RF coil and external pickup coil). No power or additional electronics are required to be on board the implanted coil. This invention is particularly useful for detecting the relaxation signals from implanted devices such as MRSw relaxation switches.

Implantation of an implantable diagnostic device allows for real-time, intermittent, or continuous monitoring of one or more analytes of interest. Implantable diagnostic devices suitable for the use with the methods of the present invention are described herein. One advantage of the methods of implantation is in conjunction with a surgical procedure, wherein a treatment site of a subject is exposed during a surgical procedure. Particular embodiments thus provide methods for implanting an implantable diagnostic device before, after, or during any type of surgery (e.g. endoscopic, laparoscopic, or open surgery). In some embodiments a surgery is conducted for a purpose other than implantation of an implantable diagnostic device.

As used herein, the terms "subject" and "patient", which in some embodiments can be a mammal, are used interchangeably. The terms "subject" and "patient" refer to an animal (e.g., a bird such as a chicken, quail or turkey, or a mammal). In some embodiments a subject is a mammal, including a non-primate (e.g., a cow, pig, horse, sheep, rabbit, guinea pig, rat, cat, dog, and mouse) and a primate (e.g., a monkey, chimpanzee and a human). In one embodiment, a subject is a non-human animal such as a farm animal (e.g., a horse, cow, pig, sheep or fish), or a companion animal (e.g., a dog, cat, guinea pig or rabbit). In a particular embodiment, the subject is a human.

It is known in the art that magnetic resonance encoding and detection can occur within a detection coil, which consists of an inductor and a capacitor, by means of a remote RF coil connected to a transceiver (transmitter and detector). Inductive coupling requires a detection coil, which is usually made of an inductor (the "coil") and a capacitor which comprises an RF circuit, and a pickup coil that consists of an inductor connected to an RF transmitter and receiver (also known as transceiver) and a magnetic field. When a detection coil is in the presence of a magnetic field, the proton within a detection volume of the detection coil can be detected by means of a coupled pickup coil. This is possible because the pickup coil and the detection coil are electromagnetically coupled, comprising a resonant circuit.

The present invention also provides for affordable means for implantable MR-relaxometry based biosensors. The nuclear magnetic resonance systems of the present invention do not require the highly homogeneous magnetic fields necessary in prior implantable MR devices to obtain magnetic resonance information, and thus allow greater flexibility in terms of the requisite instrumentation and hardware. For example, a magnetic field conveyed upon an implanted coil can be generated by an implanted miniature magnet, an external permanent magnet such as a single sided magnet, or by a Halbach array, u-magnet, toroidal magnet, or even a superconducting magnet. A dedicated magnet array can be designed to generate desired levels of magnetic field strength at the position of the implanted coil. The magnetic field strength may be in the range of 0.1 Tesla to 2 Tesla, most commonly in the range of 0.2 to 0.7 Tesla. If the field is generated by an external magnet, the configuration can be enclosed or open. Enclosed configurations typically allow for more homogenous magneto field distributions and therefore are less sensitive to the relative position of the implanted coil and the magnet. Open configurations generate less homogenous fields, but have been demonstrated to be effective for magnetic resonance and magnetic resonance measurements (P.J. Prado. Single-Sided Imaging Sensor. Magn. Reson. Imaging, 2003, 21, 397-400 - U.S. Patent 6,977,503. *System and Method for Single-Sided Magnetic Resonance Imaging* - Bluemich B, Bluemler P, Eidmann G, Guthausen A, Haken R, Schmitz U, Saito K, Zimmer G, The NMR-mouse: Construction, excitation, and applications. Magn Reson Imaging 1998; 16:479). Because the region of interest in the current implantable application is significantly smaller than that of conventional MRI diagnostics, compact magnets may be utilized to generate the magnetic field. Open magnets can be fabricated in robust, low cost, compact forms. As an example a dipole type magnet (U.S. Patent 6,977,503) generates flat surfaces of constant magnetic field outside the magnet. If the implanted coil is positioned at a know distance from the permanent magnet array, the frequency of resonance is known and magnetic resonance signals can be generated and picked up by the inductive coupling device.

In one embodiment, the invention consists of an implantable device that contains a chamber (e.g. a sample volume) containing MRSw nanoparticles. The chamber is permeable to analyte and solution from the surrounding matrix but does not allow the nanoparticles to diffuse out. The chamber is within the detection volume of a radiofrequency coil (e.g., solenoid, Helmholtz). In certain embodiments a RF coil is a solenoid. An RF coil is usually an independent circuit. In some embodiments a chamber is located within the magnetic field of one or more implantable magnets. In other embodiments a chamber is not permanently located within a magnetic field; rather a magnetic field is introduced by an external magnet positioned outside the subject. Signal from an RF coil enclosing a chamber is detected by means of an external radiofrequency coil (e.g., a pickup coil) appropriately tuned to couple (inductively coupled) with the implanted coil as known in the art. This tuning and matching characteristic of the circuit is a function of the distance between the two coils. Proper signal acquisition is also a function of the magnetic field. External magnets for use in the provided systems and methods for tuning and matching a circuit appropriately are known in the art and can be adapted for the current systems. Rollwitz WL. Agricultural Engineering 1985; 66:12; P.J. Prado. Single-Sided Imaging Sensor. Magn. Reson. Imaging, 2003, 21, 397-400; P.J. Prado. NMR Hand-Held Moisture Sensor. Magn. Reson. Imaging, 2001, 19, 506-508; P.J. Prado, B. Blümich, and U. Schmitz. One-dimensional Imaging with a Palm-Size Probe. J. Magn. Reson. 2000, 144, 200-206; U.S. Patent 6,977,503. *System and Method for Single-Sided Magnetic Resonance Imaging;* U.S. Patent 6,489,767: *Apparatus for and Method of Single-sided Magnetic Resonance Imaging with a Palm-size Probe;* Bluemich B, Bluemler P, Eidmann G, Guthausen A, Haken R, Schmitz U, Saito K, Zimmer G, The NMR-mouse: Construction, excitation, and applications. Magn Reson Imaging 1998; 16:479; MacDonald PJ. Stray field magnetic resonance imaging. Progress in NMR 1997; 30:69 -99.

As used herein, the term "nanoparticle" refers to MRSw nanoparticles, e.g. "magnetic particles," as described below.

Provided devices and methods include an implanted MR biosensor that by means of reversible magnetic resonance switch (MRSw) assays allows for the real-time sensing of analyte levels. MRSw, which undergo a switch between clustered and dispersed states due to the presence of molecular, cellular, viral analytes, can be read by a change in a MR relaxation rate. MRSw assays and sensors (e.g., provided devices) can be configured to function in a reversible fashion.

Rather than necessitating implantation of a complete RF probe or spectrometer as used in prior devices and methods, only a chamber including an RF coil is required to be implanted. Further, prior devices and methods including implanted chambers use a high-field MRI scanner; however this is costly and inconvenient or requires implantation of some combination of MR spectrometers. Using an inductively coupled coil as provided herein allows for a portable reader to be used and allows for the implantable device to be cheap and disposable. The portable reader contains a customized compact magnet array with significant lower complexity and cost than conventional superconducting counterparts. If the magnetic field is generated by an implanted permanent magnet by the implanted RF coil, the array can be even significantly smaller.

Provided devices and methods can be used for a variety of applications. Relevant applications include but are not limited to the following. A permeable sample volume (chamber) can be configured to contain the sensing volume of an implantable device (e.g., biosensor) within the sensitive region of the implanted RF coil. Measurements of relaxation rates (e.g., T2, T1, etc) with a variety of pulse sequences can be used to deduce the state of the biosensor. A biosensor can be configured to link presence and/or amount of analyte (e.g., target levels, physiological status, or biomolecular levels) to an MR parameter using one of the following methods:

Encapsulation of a smart polymer, or responsive polymers. Such polymers have been shown to be sensitive to pH, ionic strength, and specific molecular and biomolecular analytes. In these cases the hydration level, cross-link density, or other characteristic of the polymer would change in response to a change in the animal. This change in polymer state would be detected by an implanted RF coil.

The real-time sensing capabilities of the devices and methods provided herein can be used, for example, in small animals or humans to monitor response to drug or other therapies over a course of time by monitoring appropriate biomarker levels without requiring removal of body fluids, obtaining one or more specimens or sacrificing the animal. Examples of this use include, for example, monitoring drug delivery and dosing efficiencies for development or treatments such as patient dosing for chemotherapy, drug development studies, monitoring circulating active agent (e.g., insulin) levels or other biomarker levels in a non-invasive manner, real-time monitoring of foreign substances in a subject (a pharmaceutically active agent, a toxin, a poison). Also, relaxation measurements of tissue, organs, blood vessels, or other body parts of a subject can be performed.

A "magnet" as used herein can be any material or combination of materials that provides a magnetic field in at least some volume around the material. Typically, the magnet is a permanent magnet. Suitable materials include but are not limited to NdFeB, SmCo, and the like. Magnets can be configured to form new magnets, that is, magnet arrays, for example, a permanent magnet with a c-shaped yoke, a Halbach magnet (cylinder and other configurations), u-magnet, torroidal magnet and the like.

The magnets, magnet configurations and magnetic field generators of the present systems can be weak and/or provide magnetic fields that are inhomogeneous. Typically, maximum magnetic field strength values provided by the magnets and/or magnet configurations of the present invention are between about 0.2 Tesla and about 2 Tesla. More typically, they are between about 0.3 and about 1.5 Tesla. Even more typically, they are between about 0.4 and about 1.1 Tesla. Most typically, they are between about 0.45 and 0.8 Tesla. In some embodiments the magnetic field strength is less than about 2 Tesla. In certain embodiments the magnetic field strength is less than about 1.1 Tesla. In certain embodiments the magnetic field strength is less than about 0.8 Tesla.

The term "inhomogeneous" refers to magnetic fields that are lower in uniformity than those required for spectroscopy. Homogeneity is dependent on the space in which the measurement is defined. Homogeneities of the magnetic fields can range between about 10000 ppm and about 10 ppm. In some embodiments homogeneities can range between about 50 ppm and 5000 ppm. In particular embodiments, homogeneities can range between about 100 ppm and about 1000 ppm.

Also, typically, the magnetic fields employed in the present systems are effectively static, that is, they do not change substantially over time. Changes in magnetic field such as due to temperature fluctuations are considered to be not substantial, corrected by an additional controlled magnetic field, a shift in the resonance frequency, or can be taken into consideration during the magnetic resonance measurement.

The magnets in the present systems can be positioned within a subject's body and/or outside of the subject's body. In contrast to magnets for disposition outside a subject's body, magnets for complete implantation within a subject's body are preferred to be small to lessen the invasiveness of the implantation. Typically, magnets for implantation are smaller than about 2 inches in any dimension. More typically, magnets for implantation are smaller than about 1 inch in any dimension. Most typically, magnets for implantation are smaller than about 0.5 inches in any dimension. Each dimension may be independently determined.

A "magnetic field generator" as used herein, is a device that provides a magnetic field in at least some volume around the device. Typically, a magnetic field generator requires a power supply and provides the targeted magnetic field only when powered. Examples of magnetic field generators include but are not limited electromagnets with and without a metal pole (see Cardot et al. Sensors and Actuators 1994). In certain embodiments, a magnetic field generator used in the present system can be positioned within a subject's body. In other embodiments, a magnetic field generator used in the present system can be positioned outside of a subject's body. Because magnetic field generators tend to be large and complex (e.g., requiring, for example, power supply), in typical configurations a magnetic field generator will be used for disposition outside a subject's body.

One or more magnet(s) and magnetic field generator(s) for use in the present systems are selected such that the combination and position of selected components will provide a magnetic field of sufficient strength in the sample volume to allow measuring a magnetic resonance signal (e.g., a relaxometry measurement).

The magnetic field strength of a given magnet or magnetic field generator in a given volume, for example, a sample volume can be calculated and/or approximated using methods known in the art. Typically, the magnetic field strength depends on the nature of the magnet or magnetic field generator and the position of the magnet or magnetic field generator relative to the sample volume. Also, magnetic field strength of a given magnet or magnetic field generator in a sample volume can be measured using methods and devices known in the art, for example, gaussmeters, teslameters, hall effect probes, and the like. In some embodiments, magnetic field strengths within a sample volume of between about 0.2 and about 2 Tesla are sufficient to allow measuring magnetic resonance signals. In certain embodiments, magnetic field strengths within a sample volume of between about 0.3 and about 1.5 Tesla are sufficient to allow measuring magnetic resonance signals. In other embodiments, magnetic field strengths within the sample volume of between about 0.45 and about 1.5 Tesla are sufficient to allow measuring magnetic resonance signals. In still other embodiments, magnetic field strengths within the sample volume of between about 0.45 and about 1.1 Tesla are sufficient to allow measuring magnetic resonance signals.

A "probehead" as used herein is the part of the nuclear magnetic resonance system that is to be implanted, partially or completely, in a subject's body. Minimally, a probehead refers to a radiofrequency circuit that includes a radiofrequency coil wound to form a space capable of accommodating a sample volume and allowing sample to enter. In one embodiment, a probehead comprises a space capable of accommodating a sample volume and/or a port. In certain embodiments, a space capable of accommodating a sample volume and port can be, for example, a radiofrequency coil (as part of a radiofrequency circuit) wound to enclose a sample volume while providing an opening (i.e., space capable of accommodating a sample volume) to allow a sample volume to be placed within the opening. In other embodiments, a space capable of accommodating a sample volume and/or port is distinct from the opening of a radiofrequency coil but adapted to a given radiofrequency coil, for example, formed to enclose part or all of a detection volume of the radiofrequency coil. For example, a capillary for containment of a sample volume, positioned within a radiofrequency coil.

In some embodiments a radiofrequency coil is wound to enclose a volume of about 1 $\mu$l to about 10ml. In some embodiments a volume is about 1ml. In some embodiments a volume is about 500 $\mu$l. In certain embodiment a radiofrequency coil is wound to enclose a volume of less than about 100 $\mu$l. In still other embodiments a radiofrequency coil is wound to enclose a volume of less than about 10 $\mu$l are used. In still further embodiment a radiofrequency coil is wound to enclose a volume of less than about 5 $\mu$l. In particular embodiments a radiofrequency coil is wound to enclose a volume of less than about 1.6 $\mu$l. In still further particular embodiments a radiofrequency coil is wound to enclose a volume of less than about 1 $\mu$l.

A material used to form a sample volume, and, in particular, any material that may be in contact with a biological sample or tissue is typically biocompatible, and constructed of materials that allow for proper function of both the device and a host animal's biological functions. Surfaces of components of a magnetic resonance system of the present invention that will be in direct contact with tissue when implanted can be made of or coated with biocompatible material to prevent, partly or completely, biofouling and/or immune responses. Also, parts of the present systems for implantation can partially or entirely composed of biodegradable material(s). In certain embodiments a device may be coated, in whole or in one or more parts with a physiologically acceptable coating as known in the art to render an implantable device bioinert, biomimetic, or bioactive, as desired. Suitable materials include titanium, inert silicone elastomers, ceramics, glass, polymeric materials, poly-$\beta$-hydroxybutyrate (PHB) and the like. One or more sample volumes and corresponding ports can be fabricated using methods known in the art. Suitable methods include form or injection molding methods, and microfabrication methods for sample containers smaller than a few millimeters, for example, two-photon three-dimensional lithography.

A probehead may include a "housing" that encloses components of a probehead such as, for example, a radiofrequency coil and magnet. In certain embodiments at least one component of a probehead (e.g., a magnet, a magnetic field generator, a radiofrequency coil) is attached to the housing.

The probeheads of the present invention typically are for implantation within a patient (e.g., implantation in tissue, organ and/or bone of a mammal). Methods for implanting an object, whether permanently or temporarily in a mammalian body are well known in the art and such methods are adaptable for implantation of device (e.g., a probehead, a magnetic resonance system) of the present invention. Suitable probeheads are disclosed but not limited to the ones described in International Patent Application No. PCT/US08/12592, filed November 6, 2008, entitled "Small Magnet and RF Coil for Magnetic Resonance Relaxometry," (Attorney's Docket No. 1014-002), the entire teachings of which are hereby incorporated by reference.

A "port" as used herein, refers in the simplest case to an opening as provided above but can also be a structure or device that allows one or more analytes to enter and/or exit the sample volume, and may prevent other sample components to enter the sample volume. A port can be, for example, a feature or structural part of a probehead or a component thereof (e.g. radiofrequency coil) or a separate structure attached to or contained within a probehead. Furthermore, a port can be, for example, a structure with one or more openings, a semipermeable membrane, or the like. In some embodiments, a port allows analytes that lead to aggregation of magnetic particles in a sample volume to enter the sample volume and prevents sample components that would hinder the aggregation process from entering the sample volume. A port also prevents assay components, for example, magnetic particles, from leaving the device.

In certain embodiments, a probehead includes one or more separate sample volumes. In some embodiments a probehead includes between about 1 and about 100 sample volumes. In some embodiments a probehead includes between about 1 and about 10 sample volumes. In some embodiments a probehead includes two sample volumes. In certain embodiments a probehead includes one sample volume.

A probehead containing more than one sample volume may comprise a radiofrequency coil with an associated detection volume encompassing at least part of each sample volume. Alternatively, a probehead may have more than one radiofrequency coil and/or radiofrequency circuit, one for each sample volume or a subgroup of the sample volumes. In certain embodiments, a probehead comprises at least two radiofrequency coils.

For probeheads that include a plurality of separate sample volumes but only one radiofrequency coil that is employed to probe the plurality of sample volumes simultaneously, multiplexing methods may be used to distinguish the magnetic resonance signal or information from the separate sample chambers. For example, one multiplexing method that may be used is based on extracting decay constant values, for example, values of spin-spin relaxation constant $T_2$ from multi-exponential relaxation curves (see T.J. Lowery et al., Anal. Chem. (2008), 80, 1118-1123.). Relaxation data obtained using a probehead of the present invention may be fit to a decaying exponential curve defined by the following equation:

$$f(t) = \sum_{i=1}^{n} A_i \exp\left(-t \Big/ T(i)\right)$$

where $f(t)$ is the signal intensity as a function of time, $t$, $A_i$ is the amplitude coefficient for the $i$th component, and $(T)_i$ the decay constant (such as $T_2$) for the $i$th component. For relaxation phenomenon discussed here the detected signal is the sum of a discrete number of components ($i$=1,2,3,4...$n$). Such functions are called mono-, bi-, tri-, tetra- or multi-exponential, respectively. Due to the widespread need for analyzing multi-exponential processes in science and engineering, there are several established mathematical methods for rapidly obtaining estimates of $A_i$ and $(T)_i$ for each coefficient (Istratov, A. A. & Vyvenko, O. F. 1999. Exponential analysis in physical phenomena. Rev. Sci. Inst. 70 (2): 1233-1257).

Further, for probeheads that include a magnet, the magnet can be single-sided, double-sided or multi-sided. In some embodiments, the magnet is double-sided. For systems with a magnet for disposition outside the subject's body, typically, the magnet is one-sided.

Also, probeheads that include a plurality of separate sample volumes with associated radiofrequency coils for each sample volume may have each radiofrequency coil connected to a switch and associated circuitry that allows to wirelessly turn on or off each of the radiofrequency coils. This allows selectively measuring the magnetic resonance signal of a sample in a sample volume with switched on radiofrequency coil.

Alternatively, magnets or magnetic field generators providing part or all of the magnetic field in a sample volume of a probehead can be implanted separately from the probehead.

In embodiments containing multiple sample volumes, separate sample volumes can each independently have the same dimensions, different dimensions but equal volumes, or different dimensions and different volumes. Typically, each separate sample volume is between about 1 fL and about 10 mL. More typically, each separate sample volume is between about 1 pL and about 1 mL. Most typically, each separate sample volume is between about 1 μL and about 200 μL.

Implantable devices of the present invention may be used to sense and/or measure magnetic resonance signals as part of a magnetic resonance system, wherein one or more sensing reagent(s) are included within an implantable device. In particular, one or more sample volume(s) of the present invention may include one or more sensing agent. Typically, each sample volume contains one sensing agent.

A "sensing agent" as used herein is an agent that senses, responds to, or is influenced by a sample characteristic to correlate the presence and/or extent of the sample characteristic with the presence, change or magnitude of the magnetic resonance signals associated with a sample. The term "sample characteristic" as used herein refers to any chemical and/or physical property of a given sample. Suitable sample characteristics can be, but are not limited to concentration of an analyte (that is, a molecule, ion, or radical of interest in the sample), pH-value, ionic strength, hydration state (e.g., of tissue, that is, concentration of water in tissue, temperature, and the like.

Suitable sensing agents can be, but are not limited to dry reagent compositions, magnetic particles, responsive polymers, magnetic resonance contrast agents, and the like.

Dried reagent compositions that are suitable include, for example, dried biotinylated coated nanoparticles (see T.J. Lowery et al., Anal. Chem. (2008), 80, 1118-1123), for example, based on the following formulation (216 μL, 0.083 mM Fe, 10 mM PBS, 20 mg/ml dextran, pH 7.4). Dried reagent compositions can be prepared by placing a magnetic particle solution, for example, biotinylated coated nanoparticle solution into a container, for example, a container such as a glass tube, and freezing the container in a freeze dryer (e.g., VirTis freeze dryer (Gardiner, NY)), for example, at -80°C for 24h. Each of the one or more separate volumes of the sample containers may be filled by transfer of the dried reagent composition from the container that was used during freeze drying.

"Magnetic particles" as used herein, are particles that respond to or are influenced by a sample characteristic to correlate the presence and/or extent of the sample characteristic with the presence, change or magnitude of the magnetic resonance signals associated with the sample. Typically, the magnetic particles respond by aggregating or dispersing. Accordingly, "forward" (clustering or aggregation) or "reverse" (declustering or dispersion) types of assays can be employed by the methods of the present invention. Additionally, nanoassembly formation can be designed to be reversible (e.g., by temperature shift, chemical cleavage, pH shift, etc.) so that "forward" or "reverse" assays can be developed for detection of specific analytes. Forward (clustering) and reverse (declustering) types of assays can be used to detect a wide variety of biologically relevant materials. Furthermore, the spin-lattice relaxation time (T1) is considered independent of nanoparticle assembly formation and can be used to measure concentration in both nano-assembled and dispersed states within the same solution.

Also, typically, magnetic particles have an average particle size of between about 1 nm and 5μm. Magnetic particles may be paramagnetic or superparamagnetic. They can have binding moieties on their surface. The binding moieties are preferably operative to alter the aggregation of the magnetic particles as a function of the presence or concentration of the analyte. The magnetic particles may include an oxide and/or a hydroxide of Fe, Si, Sn, An, Ti, Bi, Zr, and/or Zn. In some embodiments the magnetic particles are superparamagnetic and have crystallite size from about 1 nm to about 100 nm. In some embodiments the magnetic nanoparticles have a metal oxide core of about 1 to about 25 nm, from about 3 to about 10 nm, or about 5 nm in diameter. The binding moieties may include one or more species of one or more of the following: an amino acid, a nucleic acid, an oligonucleotide, a therapeutic agent, a metabolite of a therapeutic agent, a peptide, a polypeptide, a protein, a carbohydrate, a polysaccharide, a virus, and/or bacteria. For example, in one embodiment, the binding moieties may include one, two, or more types of oligonucleotides and/or one, two, or more types of proteins. The binding moieties may be a polymer, or may be part of a polymer that is linked to, or otherwise associated with one or more of the magnetic particles. In some embodiments the binding moieties include functional groups, for example, the binding moieties may include one or more species of one or more of the following: an amino group, a carboxyl group, a sulfhydryl group, an amine group, an imine group, an epoxy group, a hydroxyl group, a thiol group, an acrylate group, and/or an isocyano group. The analyte may include one or more species of one or more of the following: a protein, a peptide, a polypeptide, an amino acid, a nucleic acid, an oligonucleotide, a therapeutic agent, a metabolite of a therapeutic agent, RNA, DNA, an antibody, an organism, a virus, bacteria, a carbohydrate, a polysaccharide, and glucose. The analyte may also include, for example, a lipid, a gas (e.g., oxygen, carbon dioxide), an electrolyte (e.g., sodium, potassium, chloride, bicarbonate, BUN, creatinine, glucose, magnesium, phosphate, calcium, ammonia, lactate), a lipoprotein, cholesterol, a fatty acid, a glycoprotein, a proteoglycan, and/or a lipopolysaccharide.

For example, magnetic particles can be adapted to respond to glycated hemoglobin. For example, amino-CLIO nanoparticles, that is, iron oxide nanoparticles coated with amino-functionalized cross-linked dextran, may be decorated with boronate compounds by standard solution-phase chemistries. The boronate compounds such as boronic acid, phenylboronic, boric acid and boronate, etc. have an affinity for HbA1c, a specific type of glycated hemoglobin designated based on its separation from other species of glycated hemoglobin. Hemoglobin is composed of four subunits, two α chains and two β chains therefore HbA1c is divalent. The divalency allows HbA1c to facilitate the boronic acid functionalized superparamagnetic iron oxide particle agglomeration. Boronate reacts with HbA1c in a sample through the cis-diol moiety of glucose bound to hemoglobin, forming a five-membered ring structure. A boronate group can be attached to a solid phase covalently or electrostatically by a variety of chemistries. Solid phases such as amino-CLIO nanoparticles can be decorated with boronate compounds by standard solution-phase chemistries. Amino-CLIO are iron oxide nanoparticles coated with amino-functionalized cross-linked dextran. The dextran polymer coating endows these nanoparticles with solubility and enabled solution-phase chemistries. Suitable boronate compounds include but are not limited to 4-carboxyphenylboronic acid, 3-nitro-5-carboxyphenylboronic acid, and m-aminophenylboronic acid (APBA).

"Nanosensors" are paramagnetic or superparamagnetic magnetic particles, typically of nanometer scale, that comprise a polymer matrix layer about a magnetic core and/or are derivatized/functionalized with binding moieties or affinity groups for a target compound or analyte. Suitable nanosensors include responsive polymer-coated magnetic nanoparticles. These nanosensors can exploit the ability of magnetic nanoparticles to dephase nuclear spins detectable by nuclear magnetic resonance (NMR), hereinafter generally exemplified as the protons of water molecules, for detection without aggregation of nanoparticles. Each nanoparticle has a polymer matrix layer which expands or contracts when exposed to an analyte and/or condition to be detected. The resulting change in nanoparticle size affects the dephasing of freely-diffusing water molecules in the vicinity of the nanoparticles, which affects one or more NMR-detectable properties. By calibrating the NMR-detected properties with known reference samples, the existence of the condition and/or analyte of interest may be detected in test samples via NMR techniques using the probeheads of the present invention.

In the case where the detected nuclei are water protons, the polymer matrix preferably takes the form of a stimuli or molecule sensitive hydrogel comprising a polymer "mesh" that is cross-linked by binding moieties that affects the volume, permeability and the proton content of the matrix as a function of a physical or chemical stimulus or a physical parameter of the analyte under study. This is accomplished by design of the matrix as a hydrophilic polymer network comprising (as pendent groups or as part of the polymer backbone) binding moieties that influence water permeability (and/or permeability of other molecules in the environment) through formation of one or more covalent or hydrogen bonds, van der Waals interactions, or physical entanglement with a component of the analyte. The presence of analyte induces a change in the crosslink density of the polymer, which leads to a change in the volume fraction of the solution occupied by the polymer. The change in cross link density also leads to a change in the diameter of the nanoparticles, which leads to a change in their diffusion time. Both diffusion time and specific volume are proportional to the $T_2$ relaxivity observed for a solution, as shown in the proportionality:

$$1/T_2 \; \alpha \; (V_p)(R^2/D)$$

where $V_p$ is the specific volume fraction of the particles in solution, R the radius of the particles, and D the diffusion constant of water. The term $R^2/D$ is equal to the diffusion time, $\tau_d$. This is the time necessary for a water molecule to diffuse past a particle, and is proportional to the extent of $T_2$ relaxation that occurs.

The binding moiety may be a chemical binder, an electroactive mediator, an electron-pair donor, and/or an electron-pair acceptor. It may contain an amino, carboxyl, sulfhydryl, amine, imine, epoxy, hydroxyl, thiol, acrylate, or isocyano group, or a mixture thereof. For example, the binding moiety may be an acetic acid moiety such as in poly(acrylic acid) for sensing pH, or phenylboronic acid for sensing the presence of diols, such as glucose Alternatively, the binding moieties are binding pairs, or binding pendants, such as antibodies that serve as cross-linkers in the presence of their cognate antigen, or antigens that serve as cross-linkers in the presence of their cognate antibodies, and which mediate the water proton flux in and out of the matrix and change in specific volume by competitive affinity reactions. This typically is accomplished as the extent of cross-linking of matrix polymer is mediated as a function of the physical parameter under study so as to control the permeability of water, including its amount and rate of translational diffusion in an out of the matrix and within the matrix volume in proximity to the magnetic particle(s). For example, the binding pairs may be a ligand binding protein such as concanavalin A bound to a low-affinity ligand such as a carbohydrate. Addition of glucose to this system would displace the low affinity ligand and change the crosslinking of the matrix. Another example is a matrix-immobilized antibody, antibody fragment, or peptide that crosslinks the matrix by binding to its matrix-immobilized antigen or target. The presence of a higher affinity analyte would lead to disruption of the cross-linked matrix and a swelling of the matrix.

The responsive matrix may comprise a matrix of material which includes one or more monomers and/or polymers. The one or more monomers and/or polymers contain functional groups that enable the binding moiety to be attached to or otherwise in stable association with the nanoparticle to form the conjugate. The polymer can be a natural polymer, a synthetic polymer, a combination of natural and synthetic polymers, shape memory polymers, block copolymers (PEO, PPO), or derivatives of each type. For example, the matrix polymer may be poly (N-isopropylacrylamide). The matrix polymer may also be (or include), for example, Poly(N-isopropylacrylamide) (PNIAAm), Poly(N,N-diethyacrylamide) (PDEAAm), P(NIAAm-co-BMA), PEO-PPO-PEO (e.g., Pluronic®), N,N-diethylaminoethyl methacrylate (DEA), 2-hydroxypropyl methacrylate (HPMA), Poly-(methacrylic acid-g-ethylen glycol), Poly(2-glucosyloxyetbyl methacrylate), Poly(N-vinyl-2pyrrolidone - co - 3-(acrylamido)phenylboronic acid), and/or N-(S)-sec-butylacrylamide. The functional groups can be any appropriate chemical functional group, e.g. carboxy, amino, or sulfhydryl groups. A specific moiety or moieties may be attached to the nanoparticle via conjugation to these groups, or by physical adsorption and/or through hydrogen bonds or van der Waals interactions. A responsive polymer matrix, through physical and/or chemical stimuli, mediates the specific volume of the polymer layer, leading to a detectable change in NMR-measurable properties such as $T_2$ relaxivity.

"Responsive polymers" (also referred to herein as "smart polymers") are polymers that are, for example, sensitive to pH, ionic strength, and/or specific molecular and/or biomolecular analytes. When utilized, the hydration level, cross-link density, or other relevant characteristic of the polymer changes in response to a change in a sample, for example, a biofluid. This change in polymer state/characteristic leads to changes in a magnetic resonance signal that can be detected by an implanted radiofrequency coil. Suitable smart polymers are known in the art, and described, for example, in Gemeinhart, RA, Chen, J, Park, H, Park, K. 2000. pH-sensitivity of fast responsive superporous hydrogels. J. Biomater. Sci. Polym. Ed. 11: 1371-1380; Murakami, Y, Maeda, M. 2005. DNA-responsive hydrogels that can shrink or swell. Biomacromolecules, 6: 2927-2929; Miyata, T, Uragami, T, Nakamae, K. 2002. Biomolecule-sensitive hydrogels. Adv Drug Deliv Rev, 54: 79-98; and Zhang, R, Bowyer, A, Eisenthal, R, Hubble, J. 2006. A smart membrane based on an antigen-responsive hydrogel. Biotechnol Bioeng.

A system of the present invention can comprise a plurality of probeheads, which can be identical or different. For example, two or more probeheads can each independently differ structurally and/or functionally. However, each probehead is adapted to form a radiofrequency resonant circuit with an external coil (also referred to herein as a "pickup coil"), and can be controlled with a control unit. A plurality of probeheads tuned at one or more than one frequency can be implanted in a given subject and/or in a number of subjects. A probehead can be implanted chronically, that is, for an extended period of time, for example, years, months or weeks, or temporarily, that is, for a short period of time, for example, days, hours or minutes.

A "radiofrequency circuit" as used herein is a circuit that includes a radiofrequency coil and may also include one or more capacitors for tuning.

Many different radiofrequency coils are known in the art. Suitable radiofrequency coils include planar coils and "whole volume" coils such as might be constructed of opposed saddle coils, solenoids, Helmholtz coils and the like. In particular embodiments, radiofrequency coils employed in the systems of the present invention include solenoids. Radiofrequency coils can be used to sense and/or detect magnetic resonance signals in an associated detection volume. Optionally, a radiofrequency coil also applies and/or emits radiofrequency signals (e.g., pulses with an associated pulse length(s)) to a sample under investigation in a corresponding detection and excitable volume that is associated with a given radiofrequency coil design as part of a magnetic resonance system. In certain embodiments a detection volume and an excitable volume are identical.

"Detection volume" as used herein refers to a volume associated with a given radiofrequency coil from which magnetic resonance signals, in principle, are detectable with the given radiofrequency coil as part of a given magnetic resonance system. "Detectable" as used herein refers to distinguishable from background noise level, that is, a magnetic resonance signal is detectable if a signal can be distinguished from background noise level with a given radiofrequency coil as part of a given magnetic resonance system. The detection volume for a given radiofrequency coil-magnetic resonance system combination can be calculated, approximated and/or measured using methods known in the art. Typically, however, it is sufficient to approximate a detection volume. For example, for a solenoid coil, typically, the detection volume is effectively, the volume enclosed within a coil, which, typically, is of about cylindrical shape. In certain embodiments a radiofrequency coil is a cylinder shape. Thus, for a solenoid a good approximation of the detection volume is the volume of the enclosed cylinder, which can be calculated very easily. Similar approximations are known in the art for other types of radiofrequency coils (see, e.g., Mispelter, J., Lupu, M., Briquet, A. "NMR Probeheads for biophysical and biomedical experiments" 2006 Imperial College Press, London.) In certain embodiments a radiofrequency coil is wound to enclose a coil volume having a shape of about cylindrical shape and the associated detection volume is effectively the volume of the cylindrical shape. In some embodiments a radiofrequency coil is positioned to have the coil volume include between about 80 percent and about 100% of the excitable volume. In still other embodiments a radiofrequency coil is positioned to have the coil volume include effectively all of the excitable volume.

An "excitable volume" as used herein is a volume of hydrogen nuclei within a sample volume that are transitioned to a higher energy state by a radiofrequency pulse of a given pulse length in the presence of a magnetic field provided by a magnet and/or magnet field generator. For example, hydrogen nuclei of a sample that are outside of the excitable volume cannot directly be excited.

An "external coil" as used herein is a coil that is suitable to couple electromagnetically (i.e., inductively) with a radiofrequency coil of a given radiofrequency circuit to form a radiofrequency resonant circuit. Typically, the external coil is connected to a control unit as part of a receiver circuit that is part of a spectrometer.

Suitable combinations of radiofrequency coil and external coil, that is, coil assemblies include but are not limited to solenoid and single-turn, solenoid and solenoid, surface coil and surface coil, surface coil and single-turn, helmholtz and solenoid, and the like. In particular embodiments, the coils are two solenoid coils.

Coupling of an external coil with an implanted radiofrequency coil allows control of an implanted radiofrequency coil without direct connection between the external coil and an implanted radiofrequency coil. It also allows inductively powering the implanted radiofrequency circuit from an external source, without the need for an implanted power supply in a subject. In certain embodiments, however, an implanted power supply, for example, included in the probehead may be used. For example, an autonomous device may be implanted which has its own internal power, and may be active while signal is detected by a passive external receiver.

In certain embodiments systems of the present invention include components, for example, one or more capacitors in a radiofrequency circuit and/or one or more capacitors in a receiving circuit to allow tuning and matching of the coil assembly to form a radiofrequency resonant circuit according to methods known in the art. In other embodiments a coil assembly may be pre-tuned.

During operation of the systems of the present invention, an external coil is inductively coupled to an implanted radiofrequency coil. Accordingly, magnetic resonance signals sensed by the implanted radiofrequency coil are effectively provided to the external coil. Likewise, control of a coupled external coil with a control unit allows inductive control of an implanted radiofrequency coil by applying a radiofrequency pulse or pulse sequence to the excitable volume of the radiofrequency coil. Typically, the excitable volume encompasses at least part of a sample volume.

A "radiofrequency pulse sequence" as used herein is a sequence of radiofrequency pulses, the pulse characteristics including a frequency of the radiofrequency pulses selected such that application of the radiofrequency pulse sequence to part or all of a sample volume leads to magnetic resonance signal that can be acquired and is associated with at least one sample contained in a sample volume. Once acquired, the raw data allows for, e.g., processing to obtain one or more nuclear magnetic resonance parameters associated with the sample. Data or NMR signal acquisition can start one or more times before, during and/or after a radiofrequency pulse sequence is applied. Typically, data or NMR signal acquisition starts between pulses of the radiofrequency pulse sequence. Standard radiofrequency pulse sequences that are suitable are known in the art, for example, a Carr-Purcell-Meiboom-Gill (CPMG) can be used if a relaxation constant $T_2$ is to be determined. Optimization of radiofrequency pulse sequences, including selection of the frequency of radiofrequency pulses in a sequence, depends on the system under investigation and is performed using procedures known in the art. Radiofrequency pulse sequences of the present invention may, for example, combine pulse sequences known in the art with one or more filter radiofrequency pulse sequence to allow determination of nuclear magnetic resonance parameters of a sample in the presence of one or more additional samples.

"Filter-based methods" as used herein, are methods that include the step of applying a filter radiofrequency pulse sequence to the samples. This is typically done as part of the pulse program, that is, at the "sample encoding" stage prior to data acquisition. Sample encoding is an inherent step in magnetic resonance based analytics and consists of inputting information into the sample in the form of radiofrequency pulses and delays. The concept of tailoring sample encoding to extract desired information from the sample is analogous to what is commonly referred to as a pulse sequence in the fields of magnetic resonance imaging and spectroscopy.

For example, one way of distinguishing multiple samples within a single coil is to apply a filter that suppresses the signal from one sample while allowing the detection of signal from another. A $T_1$-based filter represents one possibility for particle-based diagnostics. Other possible filters include those based on $T_2$, $T_1$-rho, and pulsed field gradients. It is believed that a $T_1$-filter based approach is particularly amenable to magnetic relaxation switch assays because the $1/T_1$ of a superparamagnetic nanoparticle solution, for example, comprising monocrystalline iron oxide depends linearly on the iron content of a solution and does not significantly change for analyte-free versus analyte-bound nanoparticles, as does $T_2$. Therefore, samples with high iron concentrations that have short $T_1$ values can be discriminated from those with low iron concentrations and long $T_1$ values prior to signal acquisition. This can be achieved, for example, by using a pulse sequence that combines a filter radiofrequency (RF) pulse sequence with the Carr-Purcell-Meiboom-Gill (CPMG) sequence:

$$180° - \tau_1 - 90° - \tau_{cp} - [180° \text{--} 2\tau_{cp}(acq)]_{np} - \tau_{rd} \qquad (1)$$

where 180° and 90° represent RF-pulses with 180° and 90° tip angles, $\tau_1$ is an inversion recovery delay, $\tau_{cp}$ the CPMG inter-echo delay, *(acq)* refers to signal acquisition, and $\tau_{rd}$ is the recycle delay between scans. Optimization of $\tau_{cp}$, $\tau_{rd}$, and pulse lengths is achieved as for a standard CPMG sequence, but $\tau_1$ is tuned such that the longitudinal magnetization of one or more samples is at a null value at the time of the 90° pulse that begins the CPMG readout. If a CPMG sequence is used at this point to collect signal from within an RF coil, only signal from the second sample will be present in the echo train. Likewise, if a $T_2$ measurement begins at 228 ms then only the signal from the first sample will be collected. The concept is that the signals in both chambers are manipulated and based on their properties, which can be inherent or engineered; the signal of one sample is effectively zero at the point of detection of the second sample. A single or a combination of filters can be used in conjunction with the devices and methods of the present invention. The radiofrequency pulse sequences thus allow for detection of *discrete* NMR relaxation information by means of a tailored nature of the particle assay and a one dimensional pulse sequence.

Both filter-based methods as well as signal processing-based methods allow the assignment of which signals originated from which sample chambers. In some cases such an assignment is not necessary, but in many cases assignment will enhance the quality of the end result delivered to a user. One means relies on a correlation between the volume of the sample and the amplitude coefficient in the fitting algorithm. If each sample has different relative volumes then the corresponding amplitude and $T_2$ coefficient can be appropriately assigned.

Yet another means for assigning a corrected relaxation rate to an appropriate sample is by tailoring a $T_1$ relaxation rate of each sample. The $T_1$ relaxation rate of a magnetic particle solution is inversely proportional to the concentration of magnetic particles. Therefore, two different magnetic particle solutions can be detected and assigned by means of the $T_1$-filter approach described above, based on the respective delays used to null signal from each chamber. An added advantage of this method is that different dynamic ranges can be sampled using different concentrations of magnetic particle assays due to the centers of an assay's dynamic range being roughly proportional to the magnetic particle concentration.

Filter-based methods and signal processing based methods can be combined to detect signal from more than two sample chambers, for example, four chambers. One possible application for the combination of the $T_1$-filter and signal-processing methods would be to measure a sample and reference assay for two different magnetic particle concentrations.

For a given magnet or magnet array, measuring magnetic field strengths as provided above allows to determine a one, two or three dimensional magnetic field map, that is, the functional dependence of the magnetic field strength in the selected one, two or three dimensions of space. As known in the art, the magnetic field map allows analyzing volumes inside or surrounding a magnet or magnet array in terms of magnetic field strengths and the homogeneity of the magnetic field. Typically, given suitable field strengths as provided above, at least limited homogeneity of the magnetic field, e.g., at least within a region corresponding to a suitable or desirable sample volume, is a desirable feature. Thus, an excitation frequency and bandwidth appropriate for a probehead configuration (e.g., magnet, coil configuration (e.g., a single external magnet configuration, and implantable magnet configuration)) guides the magnet and coil design, as well as the probehead configuration design.

In some embodiments probeheads include one or more magnets, wherein the magnets are in a fixed relative position and orientation to a radiofrequency coil of the radiofrequency circuit such that the sensitive volume of the radiofrequency coil encloses a volume of the magnetic field with suitable field strengths and better homogeneity (relative to other volumes that could be selected). "Sensitive volume" as used herein refers to the overlap volume between the excitable volume and the detection volume, and is the volume from which magnetic resonance signals can be detected with a radiofrequency coil. A sensitive volume is determined by a fill factor (i.e., a fraction of the detection volume of an RF coil which is filled with a sample volume). An advantage of systems including probeheads with implanted magnet(s) is that an optimized relative position and orientation between magnet(s) and a radiofrequency coil can be pre-determined and fixed and, thus, sample volume can be determined and fixed prior to implantation of the probehead in a subject's body.

In some embodiments systems have one or more magnets for disposition outside a subject's body to provide a magnetic field in a sample volume, wherein the magnet(s) is/are positioned and oriented relative to an implanted radiofrequency coil of the radiofrequency circuit such that the sensitive volume of the radiofrequency coil encloses a volume of the magnetic field with suitable field strengths and better homogeneity (relative to other volumes that could be selected). Positioning of the magnet in such systems necessarily occurs after implantation of a given probehead. Thus, in certain embodiments provided systems include means for determining a position and/or orientation of a probehead within a subject's body to optimize the position of a magnet(s) relative to the probehead. Alternatively, for example, the position and/or orientation of the magnet(s) and/or the subject body can be incrementally adjusted until a magnetic resonance signal is detected and/or maximized in strength. In still additional or alternative embodiments, a probehead may include a magnetic field detector for detecting the strength of a magnetic field within or adjacent to a sample volume, and a transmitter for transmitting a signal indicative of the strength of the magnetic field to a control unit. The control unit in such a situation includes a radio-frequency receiver for receiving a signal indicative of the strength of the magnetic field, and the position and/or orientation of a magnet can then be adjusted to increase the magnetic field strength within the sample volume.

Other embodiments include devices in the systems of the present invention to determine a position of an implanted probehead in a subject's body, and include, for example, devices that are based on x-ray telemetric location determination, near IR telemetric location determination, ultrasonic telemetric location determination, magnetic resonance telemetric location determination and the like.

A "control unit" as used herein is any device that is suitable to be connected to an external coil and comprises logic circuitry to control a radiofrequency resonant circuit to allow acquisition and processing of magnetic resonance signals sensed by a radiofrequency circuit and received by the external coil as part of the radiofrequency resonant circuit. A control unit may be custom made or a control unit of an existing magnetic resonance device, for example, a spectrometer such as a KEA spectrometer from Magritek with associated software, and then used and/or adapted for use in the magnetic resonance systems of the present invention.

In certain embodiments magnetic particles for use in the systems and methods provided herein are paramagnetic. In particular embodiments the magnetic particles are superparamagnetic. In certain embodiments magnetic particles are coated with a polymer matrix coating, for example, but not limited to, a dextran coating. In yet other embodiments magnetic particles are functionalized with one or more binding moieties that bind to one or more target analytes. Suitable binding moieties include at least one of an amino group, a carboxyl group, a sulfhydryl group, an amine group, an imine group, an epoxy group, a hydroxyl group, a thiol group, an acrylate group, or an isocyano group. Additional suitable binding moieties include at least one of an amino acid, a nucleic acid, an oligonucleotide, a therapeutic agent, a metabolite of a therapeutic agent, a peptide, a polypeptide, a protein, a carbohydrate, a polysaccharide, an antibody, a virus, or a bacterium.

The systems of the present invention measure magnetic resonance signals. The logic circuitry of the control unit can be adapted using known methods known in the art to process the sensed and acquired magnetic resonance signals, and to determine magnetic resonance relaxation parameters such as $T_1$, $T_2$, $T_{1rho}$ and the like, and other parameters such as signal intensity, signal lifetime, signal linewidth, signal integral and the like. Typically, the logic circuitry allows determination of magnetic resonance relaxation parameters. Most typically, the logic circuitry allows determination of $T_2$.

The systems of the present invention may further include a drug delivery unit, the drug delivery unit comprising one or more pharmaceutically active agents suitable for administration to a subject, and the control unit further comprising logic circuitry for controlling the drug delivery unit, the method further comprising the step of controlling the drug delivery unit with the control unit to deliver a pharmaceutically active agent from the drug delivery unit to the subject's body in response to the sensed magnetic resonance signals, and in particular, determined magnetic relaxation parameter(s).

Part or all of the components of the present systems can be part of wearing apparel. This design is particularly suitable for systems that employ probeheads with included magnet. For example, for a probehead implanted in a particular implantation side in the chest of a human, the wearing apparel, for example, a jacket would be designed to position the external coil near, and in certain embodiments, on top of the implantation side.

One embodiment of the present invention is a nuclear magnetic resonance system for measuring magnetic resonance signals from a sample contained in a sample volume *in-vivo.* The system comprises (a) a permanent magnet for disposition outside a subject's body and near the sample volume to provide a static magnetic field in the sample volume, (b) a radiofrequency circuit suitable for partial or complete implantation within a subject's body, the radiofrequency circuit comprising a radiofrequency coil to allow applying a radiofrequency pulse or pulse sequence to the sample volume in the presence of a magnetic field provided by the permanent magnet and to sense magnetic resonance signals from the sample in the sample volume, (c) an external coil for disposition outside the subject's body, the external coil being suitable to couple electromagnetically (without contact) to the radiofrequency circuit to form a radiofrequency resonant circuit, and (d) a control unit for disposition outside the subject's body, the control unit being connected to the external coil and comprising logic circuitry to control the radiofrequency resonant circuit and to allow acquisition and processing of magnetic resonance signals sensed by the radiofrequency circuit and received by the external coil as part of the radiofrequency resonant circuit.

A related embodiment of the present invention is a nuclear magnetic resonance system for measuring magnetic resonance signals from a sample contained in a sample volume *in-vivo.* The system comprises (a) a permanent magnet for disposition outside a subject's body and near a sample volume to provide a static magnetic field in the sample volume, (b) a radiofrequency circuit suitable for partial or complete implantation within a subject's body, the radiofrequency circuit comprising a radiofrequency coil to allow applying a radiofrequency pulse or pulse sequence to the sample volume in the presence of a magnetic field provided by the permanent magnet and to sense magnetic resonance signals from the sample in the sample volume, (c) an external coil for disposition outside the subject's body, the external coil being suitable to couple electromagnetically (without contact) to the radiofrequency circuit to form a radiofrequency resonant circuit, and (d) a control unit for disposition outside the subject's body, the control unit being connected to the external coil and comprising logic circuitry to control the radiofrequency resonant circuit and to allow acquisition and processing of magnetic resonance signals sensed by the radiofrequency circuit and received by the external coil as part of the radiofrequency resonant circuit to determine magnetic resonance relaxation parameters.

A further related embodiment of the present invention is a nuclear magnetic resonance system for measuring magnetic resonance signals from a sample contained in a sample volume *in-vivo.* The system comprises (a) a permanent magnet for disposition outside a subject's body and near the sample volume to provide a static magnetic field in the sample volume, (b) a radiofrequency circuit suitable for partial or complete implantation within a subject's body, the radiofrequency circuit comprising a radiofrequency coil to allow applying a radiofrequency pulse or pulse sequence to the sample volume in the presence of a magnetic field provided by the permanent magnet and to sense magnetic resonance signals from the sample in the sample volume, (c) an external coil for disposition outside the subject's body, the external coil being suitable to couple electromagnetically (without contact) to the radiofrequency circuit to form a radiofrequency resonant circuit, and (d) a control unit for disposition outside the subject's body, the control unit being connected to the external coil and comprising logic circuitry to control the radiofrequency resonant circuit and to allow acquisition and processing of magnetic resonance signals sensed by the radiofrequency circuit and received by the external coil as part of the radiofrequency resonant circuit to determine $T_2$.

Another embodiment of the present invention is a nuclear magnetic resonance system for measuring magnetic resonance signals from a sample contained in a sample volume *in-vivo.* The system comprises (a) a permanent magnet for disposition outside a subject's body and near the sample volume to provide a static magnetic field in the sample volume, (b1) a probehead suitable for partial or complete implantation within a subject's body, the probehead comprising a radiofrequency circuit comprising a radiofrequency coil wound to form a space capable of accommodating a sample volume and a port, and allowing applying a radiofrequency pulse or pulse sequence to the sample volume in the presence of a magnetic field provided by the permanent magnet and to sense magnetic resonance signals from the sample in the sample volume; wherein the sample volume contains superparamagnetic particles and the port is adapted to allow an analyte to enter the sample volume and to prevent, partly or completely, the magnetic particles from leaving the sample volume, part or effectively all of the magnetic particles aggregating in the presence of the analyte to change the magnetic resonance signal sensed by the radiofrequency circuit, (c) an external coil for disposition outside the subject's body, the external coil being suitable to couple electromagnetically (without contact) to the radiofrequency circuit to form a radiofrequency resonant circuit, and (d) a control unit for disposition outside the subject's body, the control unit being connected to the external coil and comprising logic circuitry to control the radiofrequency resonant circuit and to allow acquisition and processing of magnetic resonance signals sensed by the radiofrequency circuit and received by the external coil as part of the radiofrequency resonant circuit to determine $T_2$.

Another embodiment of the present invention is a nuclear magnetic resonance system for measuring magnetic resonance signals from a sample contained in a sample volume *in-vivo.* The system comprises (a) a permanent magnet for partial or complete implantation within a subject's body and near the sample volume to provide a static magnetic field in the sample volume, (b) a radiofrequency circuit suitable for partial or complete implantation within a subject's body, the radiofrequency circuit comprising a radiofrequency coil to allow applying a radiofrequency pulse or pulse sequence to the sample volume in the presence of a magnetic field provided by the permanent magnet and to sense magnetic resonance signals from the sample in the sample volume, (c) an external coil for disposition outside the subject's body, the external coil being suitable to couple electromagnetically (without contact) to the radiofrequency circuit to form a radiofrequency resonant circuit, and (d) a control unit for disposition outside the subject's body, the control unit being connected to the external coil and comprising logic circuitry to control the radiofrequency resonant circuit and to allow acquisition and processing of magnetic resonance signals sensed by the radiofrequency circuit and received by the external coil as part of the radiofrequency resonant circuit.

A related embodiment of the present invention is a nuclear magnetic resonance system for measuring magnetic resonance signals from a sample contained in a sample volume *in-vivo.* The system comprises (a) a permanent magnet for partial or complete implantation within a subject's body and near the sample volume to provide a static magnetic field in the sample volume, (b) a radiofrequency circuit suitable for partial or complete implantation within a subject's body, the radiofrequency circuit comprising a radiofrequency coil to allow applying a radiofrequency pulse or pulse sequence to the sample volume in the presence of a magnetic field provided by the permanent magnet and to sense magnetic resonance signals from the sample in the sample volume, (c) an external coil for disposition outside the subject's body, the external coil being suitable to couple electromagnetically (without contact) to the radiofrequency circuit to form a radiofrequency resonant circuit, and (d) a control unit for disposition outside the subject's body, the control unit being connected to the external coil and comprising logic circuitry to control the radiofrequency resonant circuit and to allow acquisition and processing of magnetic resonance signals sensed by the radiofrequency circuit and received by the external coil as part of the radiofrequency resonant circuit to determine magnetic resonance relaxation parameters.

A further related embodiment of the present invention is a nuclear magnetic resonance system for measuring magnetic resonance signals from a sample contained in a sample volume *in-vivo.* The system comprises (a) a permanent magnet for partial or complete implantation within a subject's body and near the sample volume to provide a static magnetic field in the sample volume, (b) a radiofrequency circuit suitable for partial or complete implantation within a subject's body, the radiofrequency circuit comprising a radiofrequency coil to allow applying a radiofrequency pulse or pulse sequence to the sample volume in the presence of a magnetic field provided by the permanent magnet and to sense magnetic resonance signals from the sample in the sample volume, (c) an external coil for disposition outside the subject's body, the external coil being suitable to couple electromagnetically (without contact) to the radiofrequency circuit to form a radiofrequency resonant circuit, and (d) a control unit for disposition outside the subject's body, the control unit being connected to the external coil and comprising logic circuitry to control the radiofrequency resonant circuit and to allow acquisition and processing of magnetic resonance signals sensed by the radiofrequency circuit and received by the external coil as part of the radiofrequency resonant circuit to determine $T_2$.

Another embodiment of the present invention is a nuclear magnetic resonance system for measuring magnetic resonance signals from a sample contained in a sample volume *in-vivo.* The system comprises (a) a permanent magnet for partial or complete implantation within a subject's body and near the sample volume to provide a static magnetic field in the sample volume, (b1) a probehead suitable for partial or complete implantation within a subject's body, the probehead comprising a radiofrequency circuit suitable for partial or complete implantation within a subject's body to allow applying a radiofrequency pulse or pulse sequence to the sample volume in the presence of a magnetic field provided by the permanent magnet and to sense magnetic resonance signals from the sample in the sample volume, the radiofrequency circuit comprising a radiofrequency coil wound to form a space capable of accommodating a sample volume and a port; wherein the sample volume contains superparamagnetic particles and the port is adapted to allow an analyte to enter the sample volume and to prevent, partly or completely, the magnetic particles from leaving the sample volume, part or effectively all of the magnetic particles aggregating in the presence of the analyte to change the magnetic resonance signal sensed by the radiofrequency circuit, (c) an external coil for disposition outside the subject's body, the external coil being suitable to couple electromagnetically (without contact) to the radiofrequency circuit to form a radiofrequency resonant circuit, and (d) a control unit for disposition outside the subject's body, the control unit being connected to the external coil and comprising logic circuitry to control the radiofrequency resonant circuit and to allow acquisition and processing of magnetic resonance signals sensed by the radiofrequency circuit and received by the external coil as part of the radiofrequency resonant circuit to determine $T_2$.

Another embodiment of the present invention is nuclear magnetic resonance system for measuring magnetic resonance relaxation signals from a sample contained in a sample volume *in-vivo*. The system comprises: (a) a probehead suitable for partial or complete implantation within a subject's body, the probehead comprising: a permanent magnet; a radiofrequency circuit to allow applying a radiofrequency pulse or pulse sequence to a sample volume in the presence of a magnetic field provided by the permanent magnet and to sense magnetic resonance signals from a sample in the sample volume, wherein the radiofrequency circuit comprises a radiofrequency coil wound to form a space capable of accommodating a sample volume and a port, the port allowing the sample to enter the sample volume; and a capacitor, wherein the permanent magnet is positioned near or around the radiofrequency coil to provide a magnetic field in the sample volume, the magnetic field being suitable to allow measuring magnetic resonance relaxation signals, (b) an external coil for disposition outside the subject's body, the external coil being suitable to couple electromagnetically (without contact) to the radiofrequency circuit to form a radiofrequency resonant circuit, and (d) a control unit for disposition outside the subject's body, the control unit being connected to the external coil and comprising logic circuitry to control the radiofrequency resonant circuit and to allow acquisition and processing of magnetic resonance relaxation signals sensed by the radiofrequency circuit and received by the external coil as part of the radiofrequency resonant circuit, wherein the sample volume contains magnetic particles and the port is adapted to allow an analyte to enter the sample volume and to prevent, partly or completely, the magnetic particles from leaving the sample volume, part or effectively all of the magnetic particles aggregating in the presence of the analyte to change the magnetic resonance signal sensed by the radiofrequency circuit.

Other embodiments of the present invention are the systems described in the preceding paragraphs, wherein the sample volume and the detection volume overlap in a sample-detection volume, the magnetic field within the sample-detection volume has a minimum magnetic field strength value, and the magnetic field varies by more than about 0.5%, more than about 0.75%, more than about 1%, more than about 1.25%, more than about 1.5%, more than about 1.75%, more than about 2%, more than about 2.25%, more than about 2.5 %, more than about 2.75 %, or more than about 3% within the sample-detection volume with respect to the minimum magnetic field strength value.

Additionally, other embodiments of the invention are the systems described herein, wherein the sample volume and the detection volume overlap in a sample-detection volume, the magnetic field within the sample-detection volume has a minimum magnetic field strength value of between about 0.1 Tesla and between about 0.8 Tesla and a maximum magnetic field strength value of between about 0.5 Tesla and between about 1.1 Tesla, and the magnetic field varies by more than about 0.5%, more than about 0.75%, more than about 1%, more than about 1.25%, more than about 1.5%, more than about 1.75%, more than about 2%, more than about 2.25%, more than about 2.5 %, more than about 2.75 %, or more than about 3% within the sample-detection volume with respect to the minimum magnetic field strength value.

Even further embodiments of the present invention are directed to methods of using any of the above described magnetic resonance systems with one or more probeheads implanted in one or more subjects. Typically, these methods are used to obtain *in-vivo* medical diagnostic information. More typically, these methods are used to obtain magnetic resonance relaxation information such as the above described nuclear resonance relaxation parameters.

Use of magnetic resonance systems of the present invention employing a probehead with a magnet or magnetic field generator contained within the probehead, position the magnet or magnetic field generator concurrently with implantation of the RF coil and sample volume because the probeheads are designed with a magnet or magnetic field generator positioned near or around the sample volume to provide a magnetic field in the sample volume suitable to allow measuring magnetic resonance signals. Accordingly, implanting a probehead including a magnet or magnetic field generator in a subject body automatically positions the magnet or magnetic field generator near or around the sample.

Use of magnetic resonance systems of the present invention employing a probehead that does not include a magnet or magnetic field generator requires separate positioning of the magnet or magnetic field generator. These systems may, for example, use an external magnet or an internal magnet that is not included in the probehead or separate from a radiofrequency coil. Typically, methods of using these systems require external positioning of a magnet or magnetic field generator after the radiofrequency coil or probehead has been implanted.

Positioning of an external coil may include adjusting the position of the external coil only, adjusting the position(s) of the subject body and, thus, the implanted probehead, and/or a combination of both.

One aspect of the invention provides a method for determining a magnetic resonance relaxation parameter associated with a sample contained in a sample volume *in-vivo* in a subject using a nuclear magnetic resonance system. In one embodiment, the method comprising the steps of: positioning a magnet or magnetic field generator of the nuclear magnetic resonance system near or around the sample to provide a magnetic field in the sample suitable to allow measuring magnetic resonance signals; implanting partially or completely a probehead of the nuclear magnetic resonance system within a subject's body, the probehead comprising: a radiofrequency circuit that includes a radiofrequency coil wound to form a space capable of accommodating a sample volume and a port, the port allowing a sample to enter the sample volume, wherein the sample volume contains magnetic particles and the port is adapted to allow an analyte to enter the sample volume and to prevent, partly or completely, the magnetic particles from leaving the sample volume, and at least part the magnetic particles aggregate in the presence of analyte to change a magnetic resonance signal of a sample; positioning an external coil outside the subject's body, wherein the external coil is inductively coupled to the radiofrequency circuit to form a radiofrequency resonant circuit; controlling with a control unit positioned outside the subject's body the radiofrequency circuit to apply the radiofrequency pulse or pulse sequence to the sample volume in the presence of the magnetic field; and acquiring and processing part or effectively all of the magnetic resonance signals from the sample in the sample volume sensed by the radiofrequency resonant circuit to determine a magnetic resonance relaxation parameter.

In some embodiments a control unit comprises logic circuitry for processing the magnetic resonance signals is adapted to determine one or a combination of signal intensity, signal lifetime, signal linewidth or signal integral.

In some embodiments the method comprises detecting a magnetic resonance relaxation parameter $T_1$, $T_2$, and/or $T_{1rho}$. In certain embodiments the detected magnetic resonance relaxation parameter is $T_2$.

In certain embodiments the method comprises utilizing a CPMG radiofrequency pulse.

In certain embodiments, a method comprises use of a device comprising magnetic particles wherein the magnetic particles are paramagnetic. In certain embodiments the the magnetic particles are superparamagnetic. In some embodiments at least one of the magnetic particles comprises a polymer matrix coating. In some embodiments the magnetic particles have an average particle size of between about 1 nm and 5 $\mu$m.

In certain embodiments the magnetic particles are functionalized with one or more binding moieties that bind to one or more analytes. In some embodiments the one or more binding moieties comprises at least one of an amino group, a carboxyl group, a sulfhydryl group, an amine group, an imine group, an epoxy group, a hydroxyl group, a thiol group, an acrylate group, or an isocyano group. In particular embodiments the one or more binding moieties comprises at least one of an amino acid, a nucleic acid, an oligonucleotide, a therapeutic agent, a metabolite of a therapeutic agent, a peptide, a polypeptide, a protein, a carbohydrate, a polysaccharide, a virus, or a bacteria.

In particular embodiments a sensing agent is a responsive polymer and the sample characteristic is one of pH value, ionic strength, or the concentration or presence of an analyte, wherein a characteristic of the responsive polymer changes in response to changes in the sample characteristic to change the magnetic resonance signal sensed by the radiofrequency circuit.

In one embodiment a sample contains water in a hydration state and a sample characteristic detected is the hydration state of a sample.

In certain methods, one or more provided method steps are repeated one or more times to determine one or more magnetic resonance relaxation parameters. In particular embodiments a given magnetic resonance parameter is determined two or more times to monitor a sample characteristic over time.

In particular methods, the steps further comprise administering a pharmaceutically active agent to a subject, the pharmaceutically active agent or therapeutic response changing the sample characteristic.

In particular embodiments a subject's body contains a foreign substance, the foreign substance being one or a combination of a pharmaceutically active agent, a toxin, or a poison, and the foreign substance or subject's body response to the foreign substance changing the sample characteristic.

In still other embodiments a nuclear magnetic resonance system further comprises a drug delivery unit, the drug delivery unit comprising a pharmaceutically active agent suitable for administration to the subject, and the control unit further comprising logic circuitry for controlling the drug delivery unit, the method further comprising the step of controlling the drug delivery unit with the control unit to deliver a pharmaceutically active agent from the drug delivery unit to the subject's body in response to the determined magnetic resonance relaxation parameter.

In certain embodiments a probehead is coated with a coating suitable to prevent, partly or completely, biofouling or immune responses. In certain embodiments a method comprises using a probehead is partially or completely composed of biodegradable materials.

In particular embodiments a magnet or magnetic field generator is outside the subject's body, the nuclear magnetic resonance system further comprises one or more probeheads, the probeheads being implanted in different locations in the subject's body. In certain embodiments methods of using a device comprise steps are performed one or more times for each of the probeheads.

In still other embodiments a magnet or magnetic field generator, an external coil and/or a control unit are independently or collectively part of wearing apparel for wear by the subject and/or attached to the subject's body.

In certain embodiment a radiofrequency coil has an associated detection volume and the sample volume overlaps completely with the detection volume. In some embodiments a radiofrequency coil forms all of the space accommodating the sample volume and port.

In some embodiments the magnet or magnetic field generator is single-sided. In other embodiments a magnet or magnetic field generator is two-sided.

In some embodiments the magnet or magnetic field generator is positioned outside the subject's body.

In some embodiments the probehead comprises a radiofrequency coil and a parallel capacitor and the magnet, the magnet being a permanent magnet with a position fixed relative to the position of the sample chamber. In some embodiments the probehead comprises a radiofrequency coil and a parallel capacitor, and a permanent magnet near or around the radiofrequency coil. In certain embodiments the probehead further comprises means for allowing removing and/or loading the sample volume with sensing agent, and the port is adapted to prevent, partly or completely, the sensing agent from leaving the sample volume. In particular embodiments a method utilizing a device further comprises a step of loading the sample volume with the sensing agent.

In certain embodiments the probehead further comprises a magnetic field detector for detecting the strengths of a magnetic field within or adjacent to the sample volume, and a transmitter for transmitting a signal indicative of the strength of the magnetic field; and the control unit further comprises a receiver for receiving the signal indicative of the strength of the magnetic field.

In still other embodiments a nuclear magnetic resonance system further comprises means for determining the position of the probehead within the subject's body. In certain embodiments the means for determining the position of the probehead is based on x-ray telemetric location determination, near IR telemetric location determination, ultrasonic telemetric location determination, or magnetic resonance telemetric location determination. In particular embodiments the probehead further comprises a radiofrequency identification

(RFID) emitter for emitting radiofrequency signals, and the means for determining the position of the probehead comprising (i) a radiofrequency receiver for receiving the emitted radiofrequency signals, and (ii) logic circuitry for determining the position of the sample volume from the received radiofrequency signals.

In certain embodiments a method of using a device comprises positioning an external coil by varying a position of the external coil until the presence of the resonant radiofrequency circuit is detected. In particular embodiments the external coil is part of a wand.

In some embodiments a probehead comprises a plurality of separate sample volumes and a radiofrequency coil with an associated excitable volume and detection volume, each of the sample volumes at least partly overlapping with the excitable volume and detection volume.

In certain embodiments a probehead comprises two sample volumes, and a method of using the device further comprises: controlling with a control unit positioned outside the subject's body the radiofrequency circuit to apply a readout sequence simultaneously to two samples in the respective two sample volumes in the presence of the magnetic field, wherein applying the readout sequence comprises acquiring radiofrequency signal to obtain raw data; and processing with the control unit part or effectively all of the magnetic resonance signals from the samples in the sample volumes sensed by the radiofrequency circuit to determine the magnetic resonance relaxation parameter for at least one of the two samples.

In particular embodiments, a method further comprises prior to applying the readout sequence: selecting one of the two samples as target sample and the other as filter sample; and controlling with the control unit positioned outside the subject's body the radiofrequency circuit to apply a filter radiofrequency pulse sequence simultaneously to the two samples in the respective sample volumes in the presence of a magnetic field, wherein the filter radiofrequency pulse sequence is designed to lead to a partial or complete reduction of radiofrequency signal associated with the filter sample during the step of acquiring signal.

In certain embodiments a nuclear magnetic resonance parameter is a spin-spin relaxation constant $T_2$. In particular embodiments a readout sequence is a Carr-Purcell-Meiboom-Gill (CPMG) sequence. In more particular embodiments a filter radiofrequency pulse sequence comprises a radiofrequency pulse with a tip angle of about 180 degrees. In certain embodiments a filter radiofrequency pulse sequence further comprises an inversion recovery delay that follows the radiofrequency pulse, the inversion recovery delay being selected such that the longitudinal magnetization of the at least two samples but the target sample is at about a null value when the CPMG sequence is applied.

In still additional embodiments a probehead comprises a plurality of separate sample volumes and a radiofrequency coil for each sample volume, each radiofrequency coil being connected to a switch and associated circuitry that allows to wirelessly turn on or off each of the radiofrequency coils, and each radiofrequency coil having an associated excitable volume and detection volume that at least partly overlaps with each corresponding sample volume.

In other embodiments a radiofrequency circuit comprises a radiofrequency coil and a reporter coil, any coupling of the external coil to the radiofrequency circuit being inductive and substantially via the reporter coil.

Many surgical procedures require significant follow up testing. For example, after organ transplantation, patients must be closely monitored to assess organ acceptance or rejection. Several biomarkers as well as therapeutic agents can be monitored. Levels of biomarker(s) and therapeutic agent(s) are typically measurable in interstitial fluid, blood, tissue and/or at a site of surgery. Implanting one or more diagnostic monitoring devices that can report biomarker or therapeutic agent levels in a real-time manner would allow non-invasive monitoring after surgery.

In some instances, a patient may need to wear a device that can communicate with an implanted sensor. This device may be used for acquiring data as well as data logging and transmitting of data to an appropriate health professional or medical center.

A device may have a limited or prolonged functional lifetime. In some cases a device may be completely biodegradable and in others a device may become simply biologically inert after a period of time. In limited cases, it may need to be replaced or inactivated.

A device can be coupled to a drug delivery system to afford real-time adjustments in therapeutic treatment. It can also be coupled to an early-response system or other types of data management to improve patient care.

As mentioned above, provided methods include a method of in vivo monitoring of one or more analytes in a body of a subject. In one embodiment a method comprises (a) in a surgery, surgically operating on a patient to expose a treatment site in said patient; (b) therapeutically or surgically treating an exposed treatment site; and (c) implanting an implantable diagnostic device at the exposed treatment site, said implanted diagnostic device detecting or measuring the presence and/or concentration of one or more analytes. One or more analytes can be monitored following the surgery to assess outcome of the surgery. One or more analytes can be, independently, biomarkers or therapeutic drugs. One or more analytes can be molecules present in interstitial fluid, blood, and/or tissue and/or organ(s) at or around a site of surgery. In certain embodiments a surgical operation performed can be an endoscopic surgery, a keyhole surgery, a laparoscopic surgery, or an open surgery. In certain embodiments a surgery is an organ transplant surgery (e.g., a kidney transplant, a liver transplant, a skin graft, etc).

As used herein, the term "endoscopic surgery" refers to a type of a minimally invasive diagnostic medical procedure that is used to assess the interior surfaces of an organ by inserting a tube into the body. The instrument may have a rigid or flexible tube and not only provide an image for visual inspection and photography, but also enable taking biopsies and retrieval of foreign objects.

As used herein, the term "keyhole surgery" refers to a surgery which involves very small incisions and less pain and trauma for the patient than in conventional surgery. The surgeon can see the area to be operated on by looking through a fine tube with a light on the end (known as a fiber optic light source) and carries out the operation by using special instruments inserted through the tube. Removal of gall bladder or gallstones and some operations on the prostate gland or on joints may be suitable for keyhole surgery. The operations are carried out under anesthetic.

As used herein, the term "laparoscopic surgery" refers to a surgical procedure similar to keyhole surgery but refers especially to operations performed inside the abdomen and in the peritoneum (the lining of the abdomen).

As used herein, the term "open surgery" refers to a surgical procedure which involves cutting skin and tissues so the surgeon has a direct access to the structures or organs involved. Examples of open surgery include the removal of organs, such as the gallbladder or kidney.

In another embodiment, an operation can be "minimally invasive", which refers to any surgical technique that does not require a large incision. This allows the patient to recuperate faster and with less pain.

In various embodiments, a surgery can be a diagnostic surgery, a preventive surgery, a curative surgery, or a palliative surgery.

As used herein, the term "diagnostic surgery" refers to a surgery performed for the purpose of establishing a diagnosis. Examples of diagnostic surgeries include biopsies.

As used herein, the term "preventive surgery" refers to removal by a surgeon of a tissue that does not yet contain diseased tissue (e.g., cancer cells), but has the probability of becoming diseased (e.g., cancerous) in the future.

As used herein, the term "curative surgery" refers a surgical procedure that involves a therapeutic step. Examples include appendectomy, mastectomy, and the like, implantation of artificial limbs, heart valves and the like, repairs to blood vessels, torn ligaments and the like.

As used herein, the term "palliative surgery" refers to surgeries that result in temporary improvements in patient's quality of life but do not result in elimination of the cause of disease or disorder. For example, the procedure may involve the removal of a painful primary or metastatic tumor mass such as a solitary spinal metastasis.

In one embodiment, the surgery can be a "microsurgery", which is a term referring to a surgery performed using a magnifying device to enable the surgeon to operate on tiny structures such as small arteries, nerves, the bones of the middle ear or inside the eye.

Certain embodiments include a method of determining organ transplant rejection or acceptance in a patient. The method comprises implanting an implantable diagnostic device in a subject, wherein the implanted diagnostic device is capable of detecting or measuring the presence and/or concentration of one or more analytes. A device may be implanted at a site of organ transplant before, during, or after the actual organ transplantation. Following implantation, the method includes monitoring one or more analytes using the output of the implanted diagnostic device.

In certain embodiments, one or more analytes being detected are selected from inflammation markers (e.g., cytokines (e.g., interleukins, TNF-alpha), COX-2, CRP); diabetes markers (e.g., insulin, glucose, glycosolated hemoglobin, Foxp3, KIM-1, NGAL); metabolic markers (e.g., cholesterol, triglycerides, TSG), electrolytes (e.g., potassium, calcium); cardiac markers (e.g., troponin, BNP, CK-Mb, D-dimer); viral markers (e.g., CMV, EBV, HBV, HCV, VZV, HHV-6, HHV-8); pathogens (e.g., Streptococcus pneumonia, mycobacteria), therapeutic agents (e.g., rapamycin, mycofenitol, cyclosporine, Tacrolimus, Sirolimus, coumadin, warfarin); and hormones (e.g, estradiol, FSH, LH, TSH, T3, T4). In some embodiments, the one or more analytes being detected are selected from cytokines (e.g., IL1, IL2, IL6, IL8, TNF), calcineurin, creatinine, and/or immunosuppressive therapeutic agents (e.g., cyclosporine A, Tacrolimus, Sirolimus). In one embodiment, the one or more analytes being monitored is an immunosuppressive therapeutic agent. In a particular embodiment, the one or more analytes being detected is cyclosporine A and/or Tacrolimus (FK506). In another embodiment, the one or more analytes being monitored is a cytokine (e.g., IL1, IL2, IL6, IL8, TNF).

Most transplant recipients will remain on immunosuppressive agents for the remainder of their lives in order to prevent rejection episodes. Controlled doses of immunosuppressive therapeutic agents are required to prevent over-medication, which may lead to patient susceptibility to opportunistic infection and drug toxicity effects; or under-medication, which may lead to shortened graft survival because of rejection episodes.

Cyclosporine A (CsA) and Tacrolimus (FK506) are two of the most popular immunosuppressant agents given to transplant patients today. These compounds have transformed clinical transplantation, both in terms of success and of quality-of life of the patient. The mechanism of action of these two compounds has been elucidated and extensively reviewed. See Scott et al. "Tacrolimus." Drugs 2003; 63(12):1247-97, G. Wiederrecht, E. Lam, S. Hung, M. Martin and N. Sigal; "The mechanism of action of FK-506 and Cyclosporin A." Annals of the New York Academy of Sciences, Vol 696, Issue 1 9-19, 1993; Clipstone NA, Crabtree GR. " Identification of calcineurin as a key signaling enzyme in T-lymphocyte activation." Nature 1992; 357:695-697, Schreiber SL, Crabtree GR. "The mechanism of action of cyclosporin A and FK506." Immunol Today 1992; 13:136-142.

It is well known that these immunosuppressive agents act by inhibiting calcineurin activity. Calcineurin is a phosphatase that activates many transcription factors involved in cytokine transcription; including the up regulation ofmRNA for IL-2. See Wiederrecht G, Lam E, Hung S, Martin M, Sigal N. "The mechanism of action of FK-506 and cyclosporin A." Ann NY Acad Sci USA1993; 696:9-19. By inhibiting calcineurin, these immunosuppressive agents, prevent IL-2 production, the expression of IL-2 receptors and consequently inhibit T-cell activation. See Brabletz T, Pfeuffer I, Schorr E, Siebelt F, Wirth T, Serfling E. "Transforming growth factor beta and cyclosporin A inhibit the inducible activity of the interleukin-2 gene in T cells through a noncanonical octamer-binding site." Mol Cell Biol. 1993; 13:1155-1162. The result of this inhibition is that the humoral and cellular immune responses are abolished, resulting in successful transplant acceptance.

Cytokine monitoring is also a potentially useful tool in the management of transplant patients. The utility of cytokine monitoring to predict the onset of significant rejection has been reported in the literature. Blood levels of cytokines involved in inflammation and immune activation (IL-1, IL-2, IL-6, IL-8, and TNF) have been shown to correlate with clinical outcome. It has been reported that following the levels of selected cytokines may in fact aid in the control of host versus graft rejection and may be a mechanism for the early detection of graft rejection. See Visentainer et al. "Serum cytokine levels and acute graft-versus-host disease after HLA-identical hematopoietic stem cell transplantation." Experimental Hematology, Volume 31, Issue 11, Pages 1044 - 1050 J.; Soichi et al. "Detection of IL-2 Receptor Gene Expression in Peripheral Blood from Renal Transplant Patients." Surgery Today 2001 Volume 31, Number 12, p. 1058-1064; and P.A. Corris and J. A. Kirby "A role for cytokine measurement of therapeutic monitoring of immunosuppressive drugs following lung transplantation." Clinical and Experimental Immunology 2005, 139: 176-178.

Immunosuppressive agents can be monitored, for example, by a classic immunoassay system in which an antibody:antigen interaction is detected. In the case of cyclosporine A (CsA) and tacrolimus (FK506), compounds would be coupled to a nanoparticle. Detection and quantitation of therapeutic agent levels would be done by a competitive assay format in which binding of anti-CsA or anti-Tacrolimus antibodies would compete for binding to an agent on a nanoparticle versus binding to agent present in a patient sample as it passes through the in vivo device. A binding moiety would provide a competitive interaction between bound compound and antibody and free compound in a subject sample and antibody.

Cytokines levels can also be monitored by an immunoassay. In this case an antibody specific to a cytokine of interest would be coupled to the nanoparticle. Binding of cytokine from a subject sample to the antibody-coupled nanoparticle would be detected as specimen passes through the in vivo device. A binding moiety would be an antibody specific to cytokine of interest. A binding interaction detected would be an analyte, *(i.e.* cytokine of interest) to its specific antibody coupled to a nanoparticle.

Any of a variety of antibodies raised against one or more analytes to be monitored can be employed in the methods of the present invention. For example, an anti-FK506 antibody available from United States Biological Inc., catalog no. F4150-15, can be used. In another embodiment, an anti-CsA antibody, available from Abcam, Cambridge, UK, catalog no. ab8312, can be used. Methods of conjugating antibodies to the magnetic particles are well known in the art. For example see Sun, E. et al., "Development of Nanoparticle Libraries for Biosensing", Bioconjugate Chem. 2006, 17, 109-113; and Sun, E. et al., "Clickable" Nanoparticles for Targeted Imaging, Molecular Imaging 2006, 5, 122-128. See also Hermanson, G. T., Bioconjugate Techniques, San Diego: Academic Press, 1996; and Wong, S.S., Chemistry of Protein Conjugation and Cross-Linking, CRC Press, 1991. Other reagents, including anti-cytokine antibodies and other agent binding moieties are available and can be purchased and/or generated using standard procedures known in the art.

Yet another aspect of the invention includes methods of monitoring analytes in a body of a patient. In some embodiments the method comprises: implanting an implantable magnetic resonance diagnostic device at an exposed treatment site; and detecting or measuring the presence and/or concentration of one or more analytes using magnetic resonance measurement.

In some embodiments the method comprises detecting one or more analytes to assess outcome of a surgery. In some embodiments, one or more analytes are being monitored any of before, during, or following a surgery. In certain embodiments the one or more analytes being monitored are, independently, a biomarker or a therapeutic agent. In some embodiments one or more analytes are molecules present in the interstitial fluid, the blood, or the tissue at the site of surgery.

In some embodiments a method of monitoring analytes in a body of a patient comprises implantation in conjunction with a surgery selected from an endoscopic surgery, keyhole surgery, laparoscopic surgery, or open surgery. In particular embodiments the surgery is minimally invasive.

In certain embodiments a surgery is selected from the group consisting of diagnostic surgery, preventive surgery, curative surgery, and palliative surgery. In particular embodiments the surgery is an organ transplant surgery.

In certain embodiments a method comprises using a implantable diagnostic device comprising: a sensor suitable for partial or complete implantation within the patient's body, the sensor comprising a probehead suitable for partial or complete implantation in a subject, the probehead comprising a radiofrequency circuit that includes a radiofrequency coil for applying a radiofrequency pulse sequence to the sample volume in the presence of a magnetic field provided by the magnet or magnetic field generator, wherein the coil is wound to form a space capable of accommodating a sample volume and a port, the sample volume containing magnetic particles, and the port allowing an analyte to enter the sample volume and preventing, partly or completely, the magnetic particles from leaving the sample volume, wherein an extent of aggregation of the magnetic particles is indicative of the presence or concentration of the analyte in the sample volume; a reader for disposition outside the patient's body, the reader providing results based on sensor indication of presence or concentration of the analyte in the sample volume; a magnet or magnetic field generator; and means for determining the position of the sensor within the subject's body.

In certain embodiments the method comprises using an implantable diagnostic device comprising: a conduit having an inlet for receiving the body fluid; sensor comprising a probehead suitable for partial or complete implantation in a subject, the probehead comprising a radiofrequency circuit that includes a radiofrequency coil for applying a radiofrequency pulse sequence at or near the Larmor frequency of water within the sample volume to the sample volume in the presence of a magnetic field provided to induce emission of echo radiofrequency signals from the water within the sample volume, wherein the coil is wound to form a space capable of accommodating a sample volume and a port, the sample volume containing magnetic particles, and the port allowing an analyte to enter the sample volume and preventing, partly or completely, the magnetic particles from leaving the sample volume, wherein an extent of aggregation of the magnetic particles is indicative of the presence or concentration of the analyte in the sample volume; a magnet or magnetic field generator for applying a magnetic field to the sample volume; a radio frequency coil for receiving the echo radiofrequency signals; and logic circuitry for calculation of a nuclear magnetic resonance parameter influenced by the presence or concentration of the analyte within the sample volume.

In some embodiments a method comprises using an implantable diagnostic device comprising a probehead that includes: a radiofrequency circuit comprising a radiofrequency coil wound to form a space capable of accommodating a sample volume and a port, wherein the sample volume contains a sensing agent and the port is adapted to allow the sample to enter and exit the sample volume and prevent, partly or completely, the sensing agent from exiting the sample volume, the sensing agent responding to a sample characteristic to correlate the extent of the sample characteristic with a change of the magnetic resonance relaxation parameter, and wherein the radiofrequency coil allows applying a radiofrequency pulse or pulse sequence to the sample volume in the presence of a magnetic field provided by a magnet or magnetic field generator and to sense magnetic resonance signals from the sample in the sample volume, and wherein the radiofrequency coil has an associated excitable volume and detection volume that partly or completely overlaps with the sample volume.

In particular embodiments a method further comprises positioning a magnet or magnetic field generator of the nuclear magnetic resonance device near or around the sample to provide a magnetic field in a sample suitable to allow measuring magnetic resonance signals; positioning an external coil outside the patient's body to inductively couple to the radiofrequency circuit to form a radiofrequency resonant circuit; controlling with a control unit positioned outside the patient's body the radiofrequency circuit to apply the radiofrequency pulse or pulse sequence to the sample volume in the presence of the magnetic field; and processing with the control unit part or effectively all of the magnetic resonance signals from the sample in the sample volume sensed by the radiofrequency circuit to determine the magnetic resonance relaxation parameter, wherein the control unit is connected to the external coil and comprises logic circuitry to control the radiofrequency resonant circuit and to allow acquisition and processing of magnetic resonance signals sensed by the radiofrequency circuit and received by the external coil as part of the radiofrequency resonant circuit.

In still additional embodiments a method of determining organ transplant rejection or acceptance in a patient is provided, the method comprising: implanting an implantable magnetic resonance diagnostic device in a patient, wherein the patient has undergone is undergoing, or will undergo an organ transplant surgery, the implanted diagnostic device detecting or measuring the presence and/or concentration of one or more analytes; and monitoring the one or more analytes using the output of the implanted diagnostic device in response to the organ transplant; wherein the output of the implanted device conveys information indicating whether an organ transplant is being rejected or accepted in the patient.

In certain embodiments the method comprises using an implantable diagnostic device comprising a sensor suitable for partial or complete implantation within the patient's body, the sensor comprising a radiofrequency coil for applying a radiofrequency pulse sequence to the sample volume in the presence of a magnetic field provided by the magnet or magnetic field generator, the radiofrequency coil wound to form a space capable of accommodating a sample volume and a port, the sample volume containing magnetic particles, and the port allowing the analyte to enter the sample volume and preventing, partly or completely, the magnetic particles from leaving the sample volume, the extent of aggregation of the magnetic particles being indicative of the presence or concentration of the analyte in the sample volume; a reader for disposition outside the patient's body, the reader providing results based on sensor indication of presence or concentration of the analyte in the sample volume; a magnet or magnetic field generator; and means for determining the position of the sensor within the patient's body.

In particular embodiments a diagnostic device for use in the method comprises: a conduit having an inlet for receiving the body fluid; a sensor comprising a radio frequency coil for transmitting a probe radiofrequency pulse sequence at or near the Larmor frequency of water within the sample volume to the sample volume in the presence of the magnetic field to induce emission of echo radiofrequency signals from the water within the sample volume; the radiofrequency coil wound to form a space capable of accommodating a sample volume and a port; the sample volume containing magnetic particles, and the port allowing the analyte to enter the sample volume and preventing, partly or completely, the magnetic particles from leaving the sample volume, the extent of aggregation of the magnetic particles being indicative of the presence or concentration of the analyte in the sample volume; a magnet or magnetic field generator for applying a magnetic field to the sample volume; a radio frequency coil for receiving the echo radiofrequency signals; and logic circuitry for calculation of a nuclear magnetic resonance parameter influenced by the presence or concentration of the analyte within the sample volume.

In some embodiments the method comprises using an implantable diagnostic device comprising a probehead that includes: a radiofrequency circuit comprising a radiofrequency coil wound to form a space capable of accommodating a sample volume and a port, wherein the sample volume contains a sensing agent and the port is adapted to allow the sample to enter and exit the sample volume and prevent, partly or completely, the sensing agent from exiting the sample volume, the sensing agent responding to a sample characteristic to correlate the extent of the sample characteristic with a change of the magnetic resonance relaxation parameter, and wherein the radiofrequency coil allows applying a radiofrequency pulse or pulse sequence to the sample volume in the presence of a magnetic field provided by a magnet or magnetic field generator and to sense magnetic resonance signals from the sample in the sample volume, and wherein the radiofrequency coil has an associated excitable volume and detection volume that partly or completely overlaps with the sample volume.

In still additional embodiments a method of determining organ transplant rejection or acceptance in a patient further comprises the steps of: positioning a magnet or magnetic field generator of the nuclear magnetic resonance device near or around the sample to provide a magnetic field in the sample suitable to allow measuring magnetic resonance signals; positioning an external coil outside the subject's body to inductively couple to the radiofrequency circuit to form a radiofrequency resonant circuit; controlling with a control unit positioned outside the patient's body the radiofrequency circuit to apply the radiofrequency pulse or pulse sequence to the sample volume in the presence of the magnetic field; and processing with the control unit part or effectively all of the magnetic resonance signals from the sample in the sample volume sensed by the radiofrequency circuit to determine the magnetic resonance relaxation parameter, wherein the control unit is connected to the external coil and comprises logic circuitry to control the radiofrequency resonant circuit and to allow acquisition and processing of magnetic resonance signals sensed by the radiofrequency circuit and received by the external coil as part of the radiofrequency resonant circuit.

EXEMPLIFICATION

Two nuclear magnetic resonance systems with probeheads implanted in a mammalian body **100** are schematically presented in Figure 1. The systems differ with respect to the employed magnet and, accordingly, the probehead. System A employs an internal double-sided magnet **101** whereas System B employs a single-sided external magnet **107**. Further, the probehead of System A consists of a radiofrequency circuit **105** and the magnet **101,** and System B employs the radiofrequency circuit **105** itself as the probehead. In both systems, the radiofrequency circuit **105** includes a radiofrequency coil (also referred herein as "internal coil") **104** and a capacitor **103.** Also, both systems include a receiver circuit **106** that includes an external coil **102,** the internal coil **104** defines a sample volume and port **108,** and the external coil **102** is connected to (item **109**) a control unit (not shown).

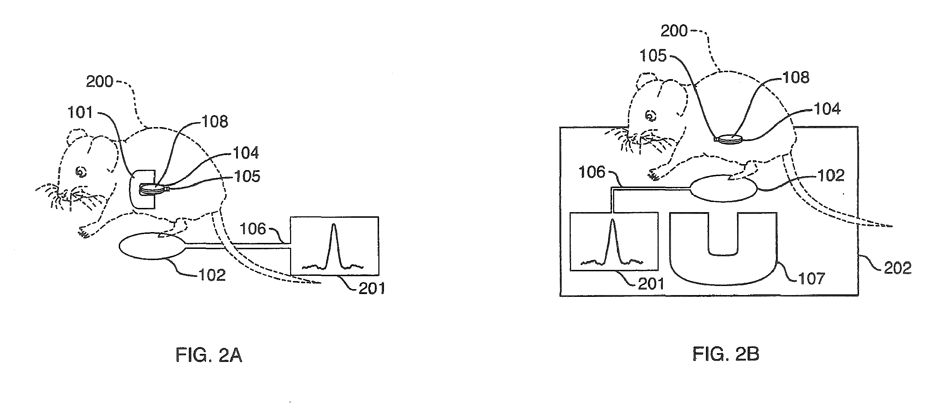

Two nuclear magnetic resonance systems with probeheads implanted in a mouse **200** are schematically presented in Figure 2. System A employs an internal double-sided magnet **101** and a radiofrequency circuit **105** as components of the probehead. System B employs a single-sided external magnet **107,** and an implanted radiofrequency circuit **105** as components of the probehead. In both systems, the radiofrequency circuit **105** includes a radiofrequency coil (also referred herein as "internal coil") **104** and a capacitor (not shown). Also, both systems include a receiver circuit **106** that includes an external coil **102.** Furthermore for both systems, the internal coil **104** defines a sample volume and port **108,** and the external coil **102** is connected to a control unit **201** via the receiver circuit **106.** In contrast to System A, System B encloses the control unit and single-sided external magnet in a separate housing **202.** An advantage of System A is that due to the absence of an external magnet, the external components of the magnetic resonance system are smaller. This allows for a mobile "wand" design for rapid in-vivo sample measurements. Additionally, the fixed relative position of internal magnet and radiofrequency coil makes tuning and matching of the system easier. An advantage of System B is that the probehead can be smaller due to the absence of an internal magnet.

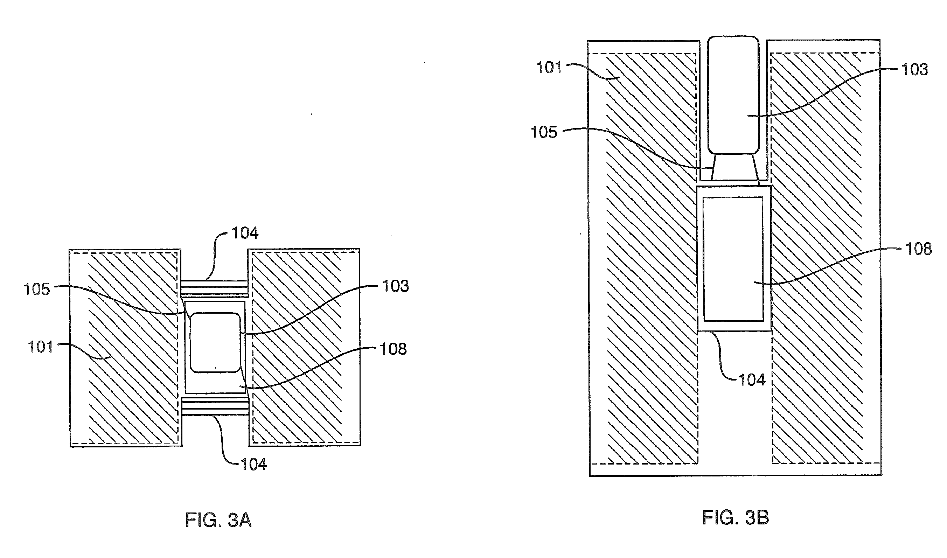

Figure 3 presents two schematic views (a top view and a side view) of a probehead that is suitable for the systems of the present invention. The probehead includes a double sided magnet **101** surrounding a radiofrequency coil **104** and a sample volume with opening (i.e. port) **108,** the radiofrequency coil **104** being part of a radiofrequency circuit **105** that also includes a capacitor **103.**

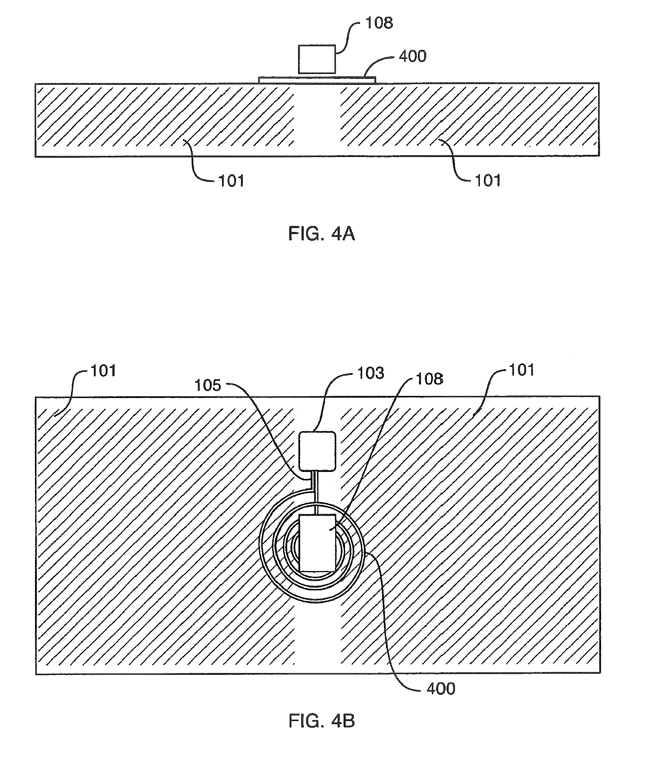

Figure 4 presents two schematic views (a top view and a side view) of a probehead that is suitable for the systems of the present invention. The probehead includes a double sided magnet **101** adjacent to a planar radiofrequency coil **400** and a sample volume with opening (i.e. port) **108,** the radiofrequency coil **104** being part of a radiofrequency circuit **105** that also includes a capacitor **103.**

The probehead design of Figure 3 provides a larger sensitive volume than the probehead design of Figure 4. On the other hand, the probehead design of Figure 4 allows easier coupling to an external coupling coil of the magnetic resonance system and provides a better fill factor.

Custom parts were machined to position the pickup coil (i.e., external coil **102**) relative to the single-sided magnet **107** such that the sensitive volume of the pickup coil contained effectively the most homogeneous region of the one-sided magnet. Two parts were machined, a surface plate **502** from cast acrylic and a coil and connector scaffold **503** from Delrin® acetal resin (DuPont). These two parts were fastened to each other with brass screws and the resulting assembly was fastened to the single-sided magnet with aluminum screws.

A single turn pickup coil **505** was fabricated from 28 gauge enamel-coated copper wire and wound using a cylindrical support as a guide. It was fastened to the Delrin® acetal resin (DuPont) scaffold using transparent adhesive tape. The ends of the enamel coated copper wire were sanded to remove the enamel coating and were soldered to a SMA-bulkhead connector that was attached to a SMA BNC cable **504** connected to a spectrometer (not shown).

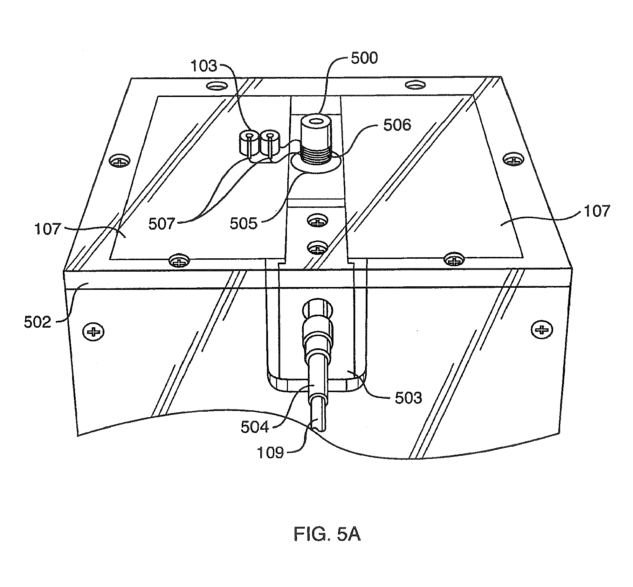

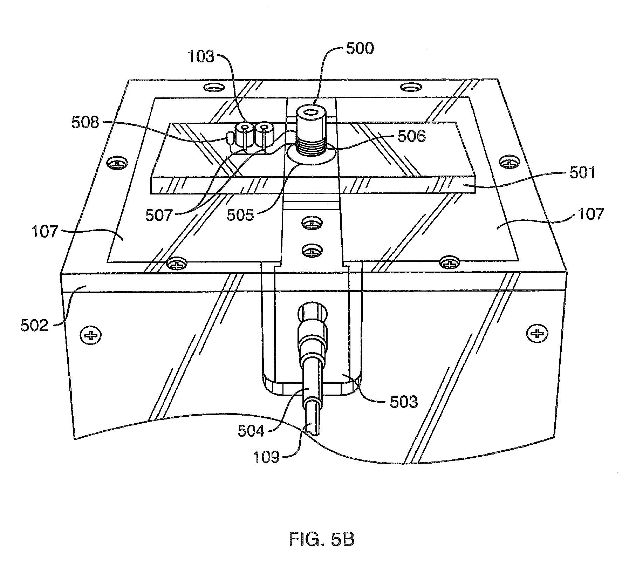

A four-turn sample coil **506** was fabricated from 28 gauge enamel-coated copper wire wound using a ~10 mm outer diameter support as a guide. Spacing between the turns was dictated by co-winding with 24 gauge enamel-coated copper wire. Adhesive tape was used to maintain the coil architecture. The ends of the coil wire were sanded to remove the enamel coated prior to attaching two non-magnetic 10-120 pF variable capacitors **507** with solder. An additional fixed capacitor (390 pF) **508** was attached to the sample coil used for sample detection 8mm from the pickup coil (see bottom part of Figure 5).

A KEA spectrometer (not shown) and Prospa software (Magritek, Wellington New Zeeland) were used for signal acquisition and processing. The sample and pickup coil combination was tuned by using the "wobb" macro and by changing the capacitance of the variable capacitors in the sample coil resonant circuit. Signal was acquired using the "cpmgadd" and "cpmgint" macros as provided by the Prospa software. Pulses were calibrated and echo times set using the standard approach.

Sample was presented to the sample coil inside of a plastic sample well obtained by modifying a 96-well sample plate. Signal was acquired from the sample coil when the center of it was positioned 3mm from the pickup coil (see top part of Figure 5) and when it was 8mm from the pickup coil (see bottom part of Figure 5) to demonstrate the capability of obtaining signal from the sample coil when it is not adjacent to the pickup coil.

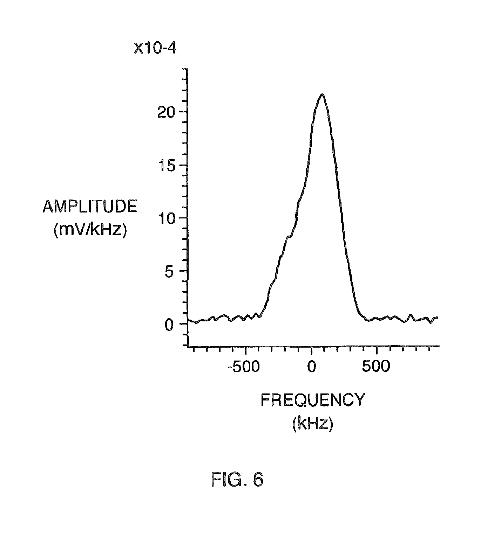

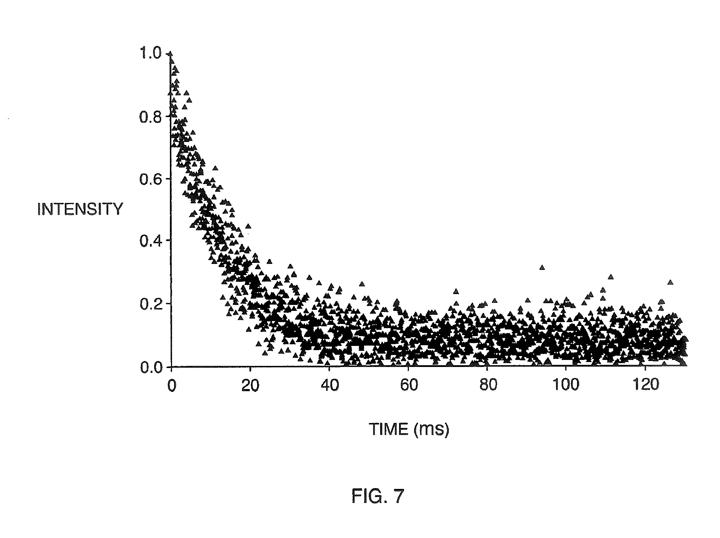

Figure 6 shows a magnitude spectrum obtained with the above described setup, wherein measurements were obtained with the pickup coil positioned 6mm from the sample coil. Figure 7 shows a $T_2$ relaxation curve obtained with the device of FIG. 5, wherein the pickup coil was positioned 6mm from the sample coil.

An additional multi-turn coil was prepared, with a sample well machined from Delrin® acetal resin (DuPont), and $T_2$ values measured from samples in an hCG assay that consisted of antibody-functionalized CLIO nanoparticles clustering in the presence of the hCG target (reference Kim, G. Y., Josephson, L., Langer, R., and Cima, M. J., Magnetic Relaxation Switch Detection of Human Chorionic Gonadotrophin, Bioconj. Chem. 2007, 18, 2024-2028.), designed to detect the presence of hCG in a device configuration similar to those described above (reference Daniel, K. D., Kim, G.

Y., Vassiliou, C. C, Jalili-Yazdi, F., Langer, R. and Cima, M. J., Multi-reservoir device for detecting a soluble cancer biomarker, Lab on a Chip 2007, 7, 1288-1293). Measurements were obtained when the center of the sample coil was about 6 mm from the pickup coil. T2 Measurements were effectively obtained from the prepared Delrin(R) acetal resin (DuPont) sample well, (data not shown.) Further, it was found that the obtained T2 resolution was significantly enhanced by entrapping the same solutions inside of a 0.5% agarose matrix (data not shown). The increase in resolution was demonstrated in hCG assays wherein no significant change in T2 values was observed in sample solutions in buffer alone, however, when embedded in 0.5% agarose a 15% (fifteen percent) decrease in T2 was observed. It is believed that the diffusion effect of water, nanoparticles, and/or nanoparticle clusters is attenuated by placing sample in a polymer matrix, enabling discrimination of clustering states of nanoparticles; and further, that additional polymer matrices would similarly improve signal resolution.

While this invention has been particularly shown and described with references to example embodiments thereof, it will be understood by those skilled in the art that various changes in form and details may be made therein without departing from the scope of the invention encompassed by the appended claims.

In some embodiments, the present invention provides:

1. A nuclear magnetic resonance system for measuring magnetic resonance signals from a sample contained in a sample volume in-vivo, the system comprising: (a) a magnet or magnetic field generator positioned to provide a magnetic field in a sample volume, the magnetic field being suitable to allow measuring magnetic resonance signal; (b) a probehead suitable for partial or complete implantation in a subject, the probehead comprising a radiofrequency circuit that includes a radiofrequency coil wound to form a space capable of accommodating a sample volume and a port, the port allowing a sample to enter the sample volume, wherein the sample volume contains magnetic particles and the port is adapted to allow an analyte to enter the sample volume and to prevent, partly or completely, the magnetic particles from leaving the sample volume; (c) an external coil for disposition outside the subject's body, wherein the external coil is suitable for inductive coupling to the radiofrequency circuit to form a radiofrequency resonant circuit; and (d) a control unit for disposition outside the subject's body, wherein the control unit is connected to the external coil and comprises logic circuitry to control the radiofrequency resonant circuit and allows acquisition and processing of magnetic resonance signal received by the radiofrequency resonant circuit.

2. A nuclear magnetic resonance system for measuring magnetic resonance relaxation signals from a sample contained in a sample volume in-vivo, the system comprising:

(a) a permanent magnet or magnetic field generator for disposition outside a subject's body and near a sample volume to provide a magnetic field in a sample volume, the magnetic field being suitable to allow measuring magnetic resonance relaxation signal;
(b) a probehead suitable for partial or complete implantation in a subject, the probehead comprising a radiofrequency circuit that includes a radiofrequency coil wound to form a space capable of accommodating a sample volume and a port, the port allowing a sample to enter the sample volume; (c) an external coil for disposition outside the subject's body, wherein the external coil is suitable for inductive coupling to the radiofrequency circuit to form a radiofrequency resonant circuit; and (d) a control unit for disposition outside the subject's body, wherein the control unit is connected to the external coil and comprises logic circuitry to control the radiofrequency resonant circuit and allows acquisition and processing of magnetic resonance relaxation signal received by the radiofrequency resonant circuit.

3. A nuclear magnetic resonance system for measuring magnetic resonance relaxation signals from a sample contained in a sample volume in-vivo, the system comprising:

(a) a probehead suitable for partial or complete implantation in a subject, the probehead comprising: (a1) a radiofrequency circuit that includes a capacitor and a radiofrequency coil wound to form a space capable of accommodating a sample volume and a port, the port allowing a sample to enter the sample volume; (a2) a permanent magnet positioned near or around the radiofrequency coil to provide a magnetic field in the sample volume, the magnetic field being suitable to allow measuring magnetic resonance relaxation signal; (b) an external coil for disposition outside the subject's body, wherein the external coil is suitable for inductive coupling to the radiofrequency circuit to form a radiofrequency resonant circuit; and (c) a control unit for disposition outside the subject's body, wherein the control unit is connected to the external coil and comprises logic circuitry to control the radiofrequency resonant circuit and allows acquisition and processing of magnetic resonance relaxation signal received by the radiofrequency resonant circuit.

4. A nuclear magnetic resonance system for measuring magnetic resonance relaxation signals from a sample contained in a sample volume in-vivo, the system comprising:

(a) a permanent magnet or magnetic field generator for disposition outside a subject's body and near a sample volume to provide a magnetic field in a sample volume, the magnetic field being suitable to allow measuring magnetic resonance relaxation signal;

(b) a probehead suitable for partial or complete implantation in a subject, the probehead comprising a radiofrequency circuit that includes a radiofrequency coil wound to form a space capable of accommodating a sample volume and a port, the port allowing a sample to enter the sample volume, wherein the sample volume contains at least one sensor particle and the port is adapted to allow an analyte to enter the sample volume and to prevent, partly or completely, the one or more sensor particles from leaving the sample volume; (c) an external coil for disposition outside the subject's body, wherein the external coil is suitable for inductive coupling to the radiofrequency circuit to form a radiofrequency resonant circuit; and (d) a control unit for disposition outside the subject's body, wherein the control unit is connected to the external coil and comprises logic circuitry to control the radiofrequency resonant circuit and allows acquisition and processing of magnetic resonance relaxation signal received by the radiofrequency resonant circuit.

5. A nuclear magnetic resonance system for measuring magnetic resonance relaxation signals from a sample contained in a sample volume in-vivo, the system comprising: (a) a probehead suitable for partial or complete implantation in a subject, the probehead comprising a probehead suitable for partial or complete implantation within a subject's body, the probehead comprising: (a1) a radiofrequency circuit that includes a capacitor and a radiofrequency coil wound to form a space capable of accommodating a sample volume and a port, the port allowing a sample to enter the sample volume, wherein the sample volume contains at least one sensor particle and the port is adapted to allow an analyte to enter the sample volume and to prevent, partly or completely, the one or more sensor particles from leaving the sample volume; (a2) a permanent magnet positioned near or around the radiofrequency coil to provide a magnetic field in the sample volume, the magnetic field being suitable to allow measuring magnetic resonance relaxation signal; (b) an external coil for disposition outside the subject's body, wherein the external coil is suitable for inductive coupling to the radiofrequency circuit to form a radiofrequency resonant circuit; and (c) a control unit for disposition outside the subject's body, wherein the control unit is connected to the external coil and comprises logic circuitry to control the radiofrequency resonant circuit and allows acquisition and processing of magnetic resonance relaxation signals received by the radiofrequency resonant circuit.

6. The system of any one of embodiments 1-5, wherein the magnetic field is effectively static.

7. The system of any one of embodiments 1-5, wherein the system is for measuring magnetic resonance relaxometry.

8. The system of any one of embodiments 1-5, wherein the radiofrequency circuit comprises a radiofrequency coil and a capacitor.

9. The system of any one of embodiments 1-5, wherein the radiofrequency coil has an associated detection volume and the sample volume overlaps completely with the detection volume.

10. The system of any one of embodiments 1-5, wherein the radiofrequency coil encompasses part of or effectively all of a sample volume and a port.

11. The system of any one of embodiments 1-5, wherein the magnet or magnetic field generator is single-sided.

12. The system of any one of embodiments 1-5, wherein the magnet or magnetic field generator is at least two-sided.

13. The system of any one of embodiments 1-2, and 4, wherein the magnet or magnetic field generator is adapted for disposition outside the subject's body.

14. The system of any one of embodiments 1-5, wherein power for operation of the radiofrequency circuit is provided via electromagnetic coupling of the external coil with the radiofrequency circuit.

15. The system of embodiment 14, wherein the external coupled power source is the sole power source for the radiofrequency circuit.

16. The system of any one of embodiments 1-5 adapted for magnetic resonance relaxometry.

17. The system of any one of embodiments 1-5, wherein the logic circuitry for processing the magnetic resonance signals is adapted to determine a magnetic resonance relaxation parameter.

18. The system of embodiment 17, wherein the magnetic resonance relaxation parameter is any of $T_1$, $T_2$, and/or $T_{1rho}$.

19. The system of any one of embodiments 1-5, wherein the logic circuitry for processing the magnetic resonance signals is adapted to determine one or a combination of signal intensity, signal lifetime, signal linewidth or signal integral.

20. The system of any one of embodiments 1-5, the radiofrequency circuit further comprising a reporter coil, wherein any coupling of the external coil to the radiofrequency circuit being inductive and substantially via the reporter coil.

21. The system of embodiment 1, wherein the probehead comprises the magnet or magnetic field generator.

22. The system of embodiment 1, wherein the radiofrequency circuit comprises a radiofrequency coil and a capacitor and the probehead comprises the magnet, the magnet being a permanent magnet with a position fixed relative to the position of the sample chamber.

23. The system of embodiment 22 further comprising (e) a detection device for determining the position of the probehead within the subject's body.

24. The system of embodiment 1, wherein the radiofrequency circuit comprises a radiofrequency coil and a capacitor, and the probehead comprises a permanent magnet near or around the radiofrequency coil.

25. The system of any one of embodiments 1 or 4-5, wherein a biosensor particle is a magnetic particle and at least part of the magnetic particles aggregate in the presence of an analyte to change a magnetic resonance signal of a sample.

26. The system of any one of embodiments 1 or 4-5, wherein the particles are paramagnetic.

27. The system of any one of embodiments 1 or 4-5, wherein the particles are superparamagnetic.

28. The system of any one of embodiments 1 or 4-5, wherein at least one of the particles comprises a polymer matrix coating.

29. The system of any one of embodiments 1 or 4-5, wherein the magnetic particles have an average particle size of between about 1 nm and 5 $\mu$m,

30. The system of any one of embodiments 1 or 4-5, wherein the magnetic particles are functionalized with one or more binding moieties that bind to one or more target analytes.

31. The system of embodiment 30, wherein at least one of the one or more binding moieties comprises at least one of an amino group, a carboxyl group, a sulfhydryl group, an amine group, an imine group, an epoxy group, a hydroxyl group, a thiol group, an acrylate group, or an isocyano group.

32. The system of embodiment 30, wherein at least one of the one or more binding moieties comprises at least one of an amino acid, a nucleic acid, an oligonucleotide, a therapeutic agent, a metabolite of a therapeutic agent, a peptide, a polypeptide, a protein, a carbohydrate, a polysaccharide, a virus, or a bacteria.

33. The system of any one of embodiments 1-5, wherein the probehead further comprises means allowing removing and/or loading a sample volume with sensor particles, and the port is adapted to allow an analyte to enter the sample volume and to prevent, partly or completely, the one or more particles from leaving the sample volume.

34. The system of any one of embodiments 1-5, wherein the probehead further comprises a magnetic field detector for detecting the strengths of the magnetic field within or adjacent to the sample volume, and a transmitter for transmitting a signal indicative of the strength of the magnetic field; and the control unit further comprises a receiver for receiving the signal indicative of the strength of the magnetic field.

35. The system of any one of embodiments 1-5 further comprising (e) a device for determining the position of the probehead within the subject's body.

36. The system of embodiment 35, wherein the device of (e) is based on x-ray telemetric location determination, near IR telemetric location determination, ultrasonic telemetric location determination, or magnetic resonance telemetric location determination.

37. The system of embodiment 35, wherein the probehead further comprises a radiofrequency identification (RFID) emitter for emitting radiofrequency signals, and the means of (e) comprising (i) a radiofrequency receiver for receiving the emitted radiofrequency signals, and (ii) logic circuitry for determining the position of the sample volume from the received radiofrequency signals.

38. The system of any one of embodiments 1-5, wherein the probehead comprises a plurality of separate sample volumes and the radiofrequency circuit comprises at least one radiofrequency coil with an associated excitable volume and detection volume for each sample volume, wherein each of the sample volumes at least partly overlap with the excitable volume and detection volume.

39. The system of any one of embodiments 1-5, wherein the probehead comprises a plurality of separate sample volumes and the radiofrequency circuit comprises a separate radiofrequency coil for each sample volume, wherein each radiofrequency coil is independently connected to a switch and associated circuitry that allows wirelessly turning on or off each of the radiofrequency coils, and wherein each radiofrequency coil has an associated excitable volume and detection volume that at least partly overlaps with each corresponding sample volume.

40. The system of any one of embodiments 1-5, further comprising at least one further probehead suitable for partial or complete implantation in a subject, the probehead comprising a radiofrequency circuit that includes a radiofrequency coil wound to form a space capable of accommodating a sample volume and a port, the port allowing a sample to enter the sample volume, wherein the sample volume contains magnetic particles and the port is adapted to allow an analyte to enter the sample volume and to prevent, partly or completely, the magnetic particles from leaving the sample volume, and at least part the magnetic particles aggregate in the presence of analyte to change a magnetic resonance signal of a sample; wherein the external coil is further adapted for inductive coupling to the radiofrequency circuit of a given probehead to form a radiofrequency resonant circuit to allow control with the control unit of the given probehead.

41. The system of any one of embodiments 1-5, wherein the sample volume and the detection volume overlap in a sample-detection volume, and the magnetic field within the sample-detection volume is inhomogeneous.

42. The system of any one of embodiments 1-5, wherein the sample volume and the detection volume overlap in a sample-detection volume, the magnetic field within the sample- detection volume has a minimum magnetic field strength value, and the magnetic field varies by more than 0.5%, more than 1%, more than 2%, and/or more than 3% within the sample-detection volume with respect to the minimum magnetic field strength value.

43. The system of any one of embodiments 1-5, wherein the magnet or magnetic field generator is positioned relative to the radiofrequency coil such that its detection volume overlaps at least partly with an excitable volume.

44. The system of any one of embodiments 1-5, wherein the sample volume and the detection volume overlap in a sample-detection volume, and the magnetic field within the sample-detection volume exhibits a maximum variation selected from between about Ippm and about 10000 ppm; between about 1 ppm and about 2000 ppm; and between about 10 ppm and about 1000 ppm.

45. The system of any one of embodiments 1-44 wherein the probehead is adapted for partial or complete implantation in a subject and for monitoring an analyte to determine outcome of a surgery.

46. The system of any one of embodiments 1-45, wherein the one or more analytes are, independently, a therapeutic agent or a biomarker.

47. The system of any one of embodiments 1-46, wherein the probehead is biodegradable or bioinert after a period of time.

48. The system of any one of embodiments 1-46, wherein the probehead is coated with a coating suitable to prevent, partly or completely, biofouling or immune response against the probehead; and/or the probehead is partially or completely composed of biodegradable materials.

49. The system of any one of embodiments 1-48, wherein the magnetic resonance system further comprises a drug delivery unit, the delivery unit comprising a pharmaceutically active agent suitable for administration to the subject, and the control unit further comprising logic circuitry for controlling the drug delivery unit, wherein the pharmaceutically active agent is delivered to the subject in response to the determined magnetic resonance signal received.

50. The system of any one of embodiments 2 or 3 wherein the sensor particle comprises a responsive polymer and a sample characteristic comprises any one of a pH value, ionic strength, or the concentration or presence of an analyte, wherein a characteristic of the responsive polymer changes in response to changes in the sample characteristic, thereby altering a magnetic resonance signal sensed by the system.

**Claims**

1. A system for measuring magnetic resonance relaxation signals from a sample contained in a sample chamber in-vivo, the system comprising:

    (a) a probehead for partial or complete implantation in a subject, the probehead comprising a radiofrequency circuit that includes a radiofrequency coil and a sample chamber comprising a port and one or more responsive polymers, the port allowing a sample to enter the sample chamber but preventing the one or more responsive polymers from leaving the sample chamber;
    (b) a magnetic field generator to provide a magnetic field in the sample chamber;
    (c) an external coil disposed external to the sample chamber and inductively coupled to the radiofrequency circuit, wherein the external coil and the radiofrequency circuit together form a radiofrequency resonant circuit; and
    (d) a control unit for disposition outside the subject's body, wherein the control unit is connected to the external coil and comprises logic circuitry to control the radiofrequency resonant circuit to allow acquisition and processing of magnetic resonance relaxation signals received by the radiofrequency resonant circuit and determination of a magnetic resonance relaxation parameter selected from $T_1$, $T_2$, and $T_{1rh0}$,
    wherein a characteristic of the responsive polymer changes in response to a change in the sample selected from a pH value, hydration state, ionic strength, or the concentration or presence of an analyte, thereby altering a magnetic resonance signal sensed by the system.

2. The system of claim 1, wherein the logic circuitry is adapted to determine one or a combination of signal intensity, signal lifetime, signal line width, or signal integral.

3. The system of claim 1 or 2, wherein the radiofrequency coil encompasses at least part of the sample chamber, and/or wherein the radiofrequency coil encompasses a detection volume that overlaps completely or in part with the sample chamber.

4. The system of any one of claims 1 to 3, wherein the magnetic field generator is single-sided and/or wherein the magnetic field generator is a permanent magnet.

5. The system of any one of claims 1 to 4, wherein the magnetic field generator is disposed outside a subject's body.

6. The system of any one of claims 1 to 4, wherein the probehead comprises the magnetic field generator.

7. The system of claim 1, wherein the sample chamber and the detection volume overlap in a sample-detection volume, and wherein the magnetic field within the sample-detection volume is inhomogeneous; the magnetic field within the sample-detection volume exhibits a maximum variation selected from between about 1 ppm and about 10000 ppm; between about 1 ppm and about 2000 ppm; and between about 10 ppm and about 1000 ppm; and/or the magnetic field within the sample-detection volume has a minimum magnetic field strength value, and the magnetic field varies by more than 0.5%, more than 1%, more than 2%, and/or more than 3% within the sample-detection volume with respect to the minimum magnetic field strength value.

8. The system of any one of claims 1 to 7, wherein the probehead further comprises a magnetic field detector for detecting the strengths of the magnetic field within or adjacent to the sample chamber, and a transmitter for transmitting a signal indicative of the strength of the magnetic field; and wherein the control unit further comprises a receiver for receiving the signal indicative of the strength of the magnetic field.

9. The system of any one of claims 1 to 8, further comprising a device for determining the position of the probehead within the subject's body.

10. The system of claim 9, wherein the device for determining the position of the probehead is based on x-ray telemetric location determination, near IR telemetric location determination, ultrasonic telemetric location determination, or magnetic resonance telemetric location determination; and/or wherein the probehead further comprises a radiofrequency identification emitter for emitting radiofrequency signals and the device for determining the position of the probehead comprises a radiofrequency receiver for receiving emitted radiofrequency signals and logic circuitry for determining the position of the sample volume from the received radiofrequency signals.

11. The system of claim 1, wherein the probehead comprises a plurality of separate sample volumes and the radiofrequency circuit comprises at least one radiofrequency coil with an associated excitable volume and detection volume for each sample volume, wherein each of the sample volumes at least partly overlap with the excitable volume and detection volume.

12. The system of claim 1, wherein the radiofrequency circuit further comprises a reporter coil for inductive coupling with the external coil.

13. The system of any one of claims 1 to 12, wherein the probehead is adapted for partial or complete implantation in a subject and for monitoring an analyte to determine outcome of a surgery.

14. The system of any one of claims 1 to 13, wherein the magnetic resonance system further comprises a drug delivery unit, the delivery unit comprising a pharmaceutically active agent for administration to the subject, and the control unit further comprising logic circuitry for controlling the drug delivery unit, wherein the pharmaceutically active agent is delivered to the subject in response to the determined magnetic resonance signal received.

15. The system of any one of claims 1 to 14, wherein one or more of the responsive polymers are coated on one or more magnetic particles.

FIG. 1B

FIG. 1A

FIG. 2B

FIG. 2A

FIG. 3B

103
108
104
101
105

FIG. 3A

103
108
104
104
105
101

108

400

101

101

FIG. 4A

101

105

103

108

101

400

FIG. 4B

FIG. 5A

FIG. 5B

FIG. 6

FIG. 7

Europäisches
Patentamt

European
Patent Office

Office européen
des brevets

## EUROPEAN SEARCH REPORT

Application Number

EP 14 19 1760

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2007/116602 A1 (LEE W D [US]) 24 May 2007 (2007-05-24) * paragraphs [0011] - [0014], [0022], [0028], [0035], [0036], [0045], [0049], [0073], [0134], [0191], [0196], [0198], [0205], [0206] *<br>----- | 1-11, 13-15 | INV.<br>G01R33/28<br>G01R33/34<br>G01R33/341<br>G01R33/38<br>G01R33/465<br>A61B5/055 |
| Y | SILVER X ET AL: "In vivo <1>H magnetic resonance imaging and spectroscopy of the rat spinal cord using an inductively-coupled chronically implanted RF coil", MAGNETIC RESONANCE IN MEDICINE WILEY USA, vol. 46, no. 6, December 2001 (2001-12), pages 1216-1222, XP002525445, ISSN: 0740-3194 * page 1216 - page 1218 *<br>----- | 1-11, 13-15 | A61B5/00<br><br>ADD.<br>G01R33/36 |
| Y | SCHNALL M D ET AL: "Wireless implanted magnetic resonance probes for in vivo NMR", JOURNAL OF MAGNETIC RESONANCE, ACADEMIC PRESS, LONDON, GB, vol. 68, no. 1, 1 June 1986 (1986-06-01), pages 161-167, XP023958675, ISSN: 0022-2364, DOI: 10.1016/0022-2364(86)90326-4 [retrieved on 1986-06-01] * the whole document *<br>----- | 1-11, 13-15 | |

TECHNICAL FIELDS SEARCHED (IPC)

G01R

The present search report has been drawn up for all claims

| Place of search | Date of completion of the search | Examiner |
|---|---|---|
| Munich | 24 April 2015 | Skalla, Jörg |

**ANNEX TO THE EUROPEAN SEARCH REPORT
ON EUROPEAN PATENT APPLICATION NO.**

EP 14 19 1760

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2015

| Patent document cited in search report | Publication date | Patent family member(s) | Publication date |
|---|---|---|---|
| US 2007116602 A1 | 24-05-2007 | AU 2006284832 A1 | 08-03-2007 |
| | | CA 2620861 A1 | 08-03-2007 |
| | | EP 1932009 A2 | 18-06-2008 |
| | | JP 5295769 B2 | 18-09-2013 |
| | | JP 2009506345 A | 12-02-2009 |
| | | US 2007116602 A1 | 24-05-2007 |
| | | US 2009134869 A1 | 28-05-2009 |
| | | US 2011018538 A1 | 27-01-2011 |
| | | US 2011020787 A1 | 27-01-2011 |
| | | US 2011020788 A1 | 27-01-2011 |
| | | US 2011020953 A1 | 27-01-2011 |
| | | US 2011021374 A1 | 27-01-2011 |
| | | US 2012107839 A1 | 03-05-2012 |
| | | US 2012313639 A1 | 13-12-2012 |
| | | US 2013229179 A1 | 05-09-2013 |
| | | WO 2007027843 A2 | 08-03-2007 |

EPO FORM P0459

For more details about this annex : see Official Journal of the European Patent Office, No. 12/82

## REFERENCES CITED IN THE DESCRIPTION

**Patent documents cited in the description**

- US 61008646 A
- US 61008669 A
- US 61127514 A
- US 20030092029 A, Josephson

- US 6977503 B
- US 6489767 B
- US 0812592 W

**Non-patent literature cited in the description**

- **PEREZ et al.** Use of Magnetic Nanoparticles as Nanosensors to Probe for Molecular Interactions. *Chem Bio Chem,* 2004, vol. 5, 261-264
- **P.J. PRADO.** Single-Sided Imaging Sensor. *Magn. Reson. Imaging,* 2003, vol. 21, 397-400
- **BLUEMICH B ; BLUEMLER P ; EIDMANN G ; GUTHAUSEN A ; HAKEN R ; SCHMITZ U ; SAITO K ; ZIMMER G.** The NMR-mouse: Construction, excitation, and applications. *Magn Reson Imaging,* 1998, vol. 16, 479
- **ROLLWITZ WL.** *Agricultural Engineering,* 1985, vol. 66, 12
- **P.J. PRADO.** Single-Sided Imaging Sensor. *Magn. Reson. Imaging,* 2003, vol. 21, 397-400
- **P.J. PRADO.** NMR Hand-Held Moisture Sensor. *Magn. Reson. Imaging,* 2001, vol. 19, 506-508
- **P.J. PRADO ; B. BLÜMICH ; U. SCHMITZ.** One-dimensional Imaging with a Palm-Size Probe. *J. Magn. Reson.,* 2000, vol. 144, 200-206
- **BLUEMICH B ; BLUEMLER P ; EIDMANN G ; GUTHAUSEN A ; HAKEN R ; SCHMITZ U ; SAITO K ; ZIMMER G.** The NMR-mouse: Construction, excitation, and applications. *Magn Reson Imaging,* 1998, vol. 16, 479
- **MACDONALD PJ.** Stray field magnetic resonance imaging. *Progress in NMR,* 1997, vol. 30, 69-99
- **CARDOT et al.** *Sensors and Actuators,* 1994
- **T.J. LOWERY et al.** *Anal. Chem.,* 2008, vol. 80, 1118-1123
- **ISTRATOV, A. A. ; VYVENKO, O. F.** Exponential analysis in physical phenomena. *Rev. Sci. Inst.,* 1999, vol. 70 (2), 1233-1257
- **GEMEINHART, RA ; CHEN, J ; PARK, H ; PARK, K.** pH-sensitivity of fast responsive superporous hydrogels. *J. Biomater. Sci. Polym. Ed.,* 2000, vol. 11, 1371-1380
- **MURAKAMI, Y ; MAEDA, M.** DNA-responsive hydrogels that can shrink or swell. *Biomacromolecules,* 2005, vol. 6, 2927-2929

- **MIYATA, T ; URAGAMI, T ; NAKAMAE, K.** Biomolecule-sensitive hydrogels. *Adv Drug Deliv Rev,* 2002, vol. 54, 79-98
- **ZHANG, R ; BOWYER, A ; EISENTHAL, R ; HUBBLE, J.** A smart membrane based on an antigen-responsive hydrogel. *Biotechnol Bioeng.,* 2006
- **MISPELTER, J. ; LUPU, M. ; BRIQUET, A.** NMR Probeheads for biophysical and biomedical experiments. Imperial College Press, 2006
- **SCOTT et al.** Tacrolimus. *Drugs,* 2003, vol. 63 (12), 1247-97
- The mechanism of action of FK-506 and Cyclosporin A. **G. WIEDERRECHT ; E. LAM ; S. HUNG ; M. MARTIN ; N. SIGAL.** Annals of the New York Academy of Sciences. 1993, vol. 696
- **CLIPSTONE NA ; CRABTREE GR.** Identification of calcineurin as a key signaling enzyme in T-lymphocyte activation. *Nature,* 1992, vol. 357, 695-697
- **SCHREIBER SL ; CRABTREE GR.** The mechanism of action of cyclosporin A and FK506. *Immunol Today,* 1992, vol. 13, 136-142
- **WIEDERRECHT G ; LAM E ; HUNG S ; MARTIN M ; SIGAL N.** The mechanism of action of FK-506 and cyclosporin A. *Ann NY Acad Sci USA,* 1993, vol. 696, 9-19
- **BRABLETZ T ; PFEUFFER I ; SCHORR E ; SIEBELT F ; WIRTH T ; SERFLING E.** Transforming growth factor beta and cyclosporin A inhibit the inducible activity of the interleukin-2 gene in T cells through a noncanonical octamer-binding site. *Mol Cell Biol.,* 1993, vol. 13, 1155-1162
- **VISENTAINER et al.** Serum cytokine levels and acute graft-versus-host disease after HLA-identical hematopoietic stem cell transplantation. *Experimental Hematology,* vol. 31 (11), 1044-1050
- **SOICHI et al.** Detection of IL-2 Receptor Gene Expression in Peripheral Blood from Renal Transplant Patients. *Surgery Today,* 2001, vol. 31 (12), 1058-1064

- **P.A. CORRIS ; J. A. KIRBY.** A role for cytokine measurement of therapeutic monitoring of immunosuppressive drugs following lung transplantation. *Clinical and Experimental Immunology,* 2005, vol. 139, 176-178
- **SUN, E. et al.** Development of Nanoparticle Libraries for Biosensing. *Bioconjugate Chem.,* 2006, vol. 17, 109-113
- **SUN, E. et al.** Clickable. *Nanoparticles for Targeted Imaging, Molecular Imaging,* 2006, vol. 5, 122-128
- **HERMANSON, G. T.** Bioconjugate Techniques. Academic Press, 1996
- **WONG, S.S.** Chemistry of Protein Conjugation and Cross-Linking. CRC Press, 1991
- **KIM, G. Y ; JOSEPHSON, L. ; LANGER, R. ; CIMA, M. J.** Magnetic Relaxation Switch Detection of Human Chorionic Gonadotrophin. *Bioconj. Chem.,* 2007, vol. 18, 2024-2028
- **DANIEL, K. D. ; KIM, G. Y. ; VASSILIOU, C. C ; JALILI-YAZDI, F. ; LANGER, R. ; CIMA, M. J.** Multi-reservoir device for detecting a soluble cancer biomarker. *Lab on a Chip,* 2007, vol. 7, 1288-1293